# EUROPEAN PATENT SPECIFICATION

(11) **EP 3 966 574 B1**
(45) Date of publication and mention of the grant of the patent: **28.08.2024**
(21) Application number: 20802683.1
(22) Date of filing: 07.05.2020
(51) Int. Cl.: G01N 33/74, B82Y 15/00

(54) **NOVEL LIGAND ASSAYS**
NEUARTIGE LIGANDENTESTS
NOUVEAUX DOSAGES DE LIGANDS

(30) Priority: 07.05.2019 NZ 19753245
(43) Date of publication of application: 16.03.2022
(73) Proprietor: InsituGen Limited, Dunedin 9016 (NZ)
(72) Inventor: HEATHER, Alison Kay, Dunedin, 9013 (NZ); SOWERBY, Stephen John, Dunedin, 9076 (NZ)
(74) Representative: J A Kemp LLP
(86) International application number: PCT/NZ2020/050045
(87) International publication number: WO 2020/226512

(56) References cited:
- EP-A1- 3 704 492
- US-A- 5 945 279
- STEVEN LUKMAN ET AL: "Hybrid Sensor Using Gold Nanoparticles and Conjugated Polyelectrolytes for Studying Sequence Rule in Protein–DNA Interactions", APPLIED MATERIALS & INTERFACES, vol. 5, no. 23, 22 November 2013 (2013-11-22), US, pages 12725 - 12734, XP055760285, ISSN: 1944-8244, DOI: 10.1021/am404120q
- MARY SZATKOWSKI OZERS ET AL: "Equilibrium Binding of Estrogen Receptor with DNA Using Fluorescence Anisotropy", JOURNAL OF BIOLOGICAL CHEMISTRY, vol. 272, no. 48, 28 November 1997 (1997-11-28), US, pages 30405 - 30411, XP055760286, ISSN: 0021-9258, DOI: 10.1074/jbc.272.48.30405
- STEVEN LUKMAN, KHIN MOH MOH AUNG, JIE LIU, BIN LIU, XIAODI SU: "Hybrid Sensor Using Gold Nanoparticles and Conjugated Polyelectrolytes for Studying Sequence Rule in Protein-DNA Interactions", APPLIED MATERIALS & INTERFACES, vol. 5, no. 23, 11 December 2013 (2013-12-11), US, pages 12725 - 12734, XP055760285, ISSN: 1944-8244, DOI: 10.1021/am404120q
- MARY SZATKOWSKI OZERS, JOHN J. HILL, KERRY ERVIN, JENNIFER R. WOOD, ANN M. NARDULLI, CATHERINE A. ROYER, JACK GORSKI: "Equilibrium Binding of Estrogen Receptor with DNA Using Fluorescence Anisotropy", JOURNAL OF BIOLOGICAL CHEMISTRY, vol. 272, no. 48, 1997, pages 30405 - 30411, XP055760286, DOI: 10.1074/jbc.272.48.30405
- M. JEYAKUMAR, PAUL WEBB, JOHN D. BAXTER, THOMAS S. SCANLAN, JOHN A. KATZENELLENBOGEN: "Quantification of Ligand-Regulated Nuclear Receptor Corepressor and Coactivator Binding, Key Interactions Determining Ligand Potency and Efficacy for Thyroid Hormone Receptor", BIOCHEMISTRY, vol. 47, no. 28, 2008, pages 7465 - 7476, XP055760288, DOI: 10.1021/bi800393u

## Description

### TECHNICAL FIELD

The invention relates generally to assays, methods and test kits for detection of a ligand in a sample. In particular, the present invention provides assays, methods and test kits to screen a test sample for the presence of a ligand characterized by its ability to form a complex with a steroid hormone receptor and elicit a steroid hormone specific genomic response.

### BACKGROUND OF THE INVENTION

The detection of ligands capable of eliciting a steroid hormone genomic response is important in many areas of biochemistry, molecular biology and medicine. Such ligands include endogenous steroids, exogenous steroids, non-steroidal and synthetic molecules. For example, the determination of total hormone bioactivity in serum or plasma is important for monitoring human health related conditions including aging, perimenopause, menopause, hypoandrogenism, hyperandrogenism, hormone replacement therapy, endocrine cancers including breast and prostate cancers, other hormone related conditions such as osteoporosis and liver toxicity, irregular menstruation, polycystic ovary syndrome, disorders of sexual development and infertility. Conventional detection methods for androgenic/estrogenic and antiandrogenic/antiestrogenic molecules provide no information about hormone biological activity. Hormone biological activity is an important measurement for understanding underlying mechanisms that are driving health conditions so that appropriate treatments/interventions can be implemented.

The detection of hormonal bioactivity in samples is also important for monitoring illicit human and animal performance enhancement, injury cover-up, supplement and food adulteration, growth promoters in dairy industry and environmental pollutants. Measuring hormonal bioactivity provides information about contaminants and/or adulterants that are likely to modulate endocrine pathways in the body, thereby affecting human health.

Ligands that elicit a steroid hormone genomic response first activate steroid hormone receptor proteins by forming a complex with them in the cytoplasm of eukaryote cells to form an activated receptor protein. The ligand displaces co-factors on the receptor protein, which then exposes DNA binding motifs. The activated receptor protein dimerises with a second activated receptor protein and translocates to the nucleus if need be to interact with DNA by binding as a dimer to a specific nucleotide sequence called a response element. In normal biological function, the assemblage of ligand-activated steroid hormone receptor proteins bound to a response element regulates gene expression by enhancing or repressing the initiation of RNA polymerase II mediated transcription. RNA polymerase II is a multi-subunit holoenzyme that assembles to catalyse RNA transcription by polymerising nucleotide triphosphates against a DNA template.

The steroid hormone genomic response is induced by ligands that bind to steroid hormone receptor proteins and receptor-specific response elements for example androgen receptor (AR) and the androgen response element (ARE), estrogen receptor-a (ER-α), estrogen receptor-β (ER-β) and the estrogen response element (ERE), glucocorticoid receptor (GR) and the glucocorticoid response element (GRE), mineralocorticoid receptor (MR) and the mineralocorticoid response element (MRE), progesterone receptor-A (PR-A), progesterone receptor-B (PR-B) and the progesterone response element (PRE).

However, not all ligands that bind to steroid hormone receptor proteins elicit a steroid hormone genomic response. Some ligands elicit a non-genomic response that is characterised by second messenger signalling, such as G-protein activation. Such non-genomic responses occur within seconds to minutes of ligand binding, and are not a classical steroid hormone response.

A common way to detect the presence of a ligand in a sample is to measure it directly in that sample. However, samples are often complex mixtures of molecules and typically require a complicated process of preparation for analysis. Detecting the presence of a ligand(s) in a sample typically relies on processes such as liquid or gas chromatography to separate the molecular species from a complex mixture into fractions of relatively pure composition and then analyse each fraction with a structure-sensitive method such as mass spectrometry. More than 100 ligands can be tested in any one sample using this approach. Automated purification systems, gas or liquid chromatograms, and mass spectrometers are costly and technically complicated laboratory instruments that must be continually calibrated and operated by trained technicians in order to produce reliable results. Another disadvantage is that some ligands may be rendered biologically inactive by interaction with proteins such as sex hormone binding globulin or serum albumin and this methodology does not distinguish between biologically active and inactive fractions of ligands. Also, the process of ionization can lead to disintegration of some steroid molecules such that they cannot be measured using such methodologies. Additionally, this methodology does not provide information about the total biological activity of a sample from multiple ligands when all known ligands cannot be identified or where ligands may be identified it is not known if the activity would be additive, synergistic, or even competitive. Furthermore, prior knowledge of the molecular structure of the ligand(s) and its associated metabolite(s) due to the biological metabolism of the ligand(s) is required to achieve reliable identification of the presence of ligand(s) in the sample.

Another common way to detect the presence of a steroid hormone ligand in a sample is to use biological assays based on immunological techniques, such as radioimmunoassay and enzyme-linked immunosorbent assay. A limitation of immunological techniques is the requirement for antibody molecules to detect the ligands directly or the ligands bound to sex hormone binding globulin. Immunological assays lack reproducibility due to the high degree of variability in the antibody molecules produced by different manufactures of the assays.

To overcome limitations with detection of ligands in a sample, cell-based steroid hormone assays have been developed in which ligands bind to steroid hormone receptor proteins and elicit a steroid hormone genomic response. In these assays, the presence of a ligand in a sample is detected after the steroid hormone receptor is activated and increases RNA polymerase II transcription of a reporter gene, which is then translated into a protein. Most commonly, the reporter gene encodes a fluorescent protein (such as GFP) or an enzyme that will induce a light or colorimetric reaction in the presence of specific substrate.

However, there are significant limitations associated with these cell-based assays in that they require specialised equipment and expertise to maintain living cell cultures. This increases the cost of cell-based testing and reduces high throughput application of these methods. Additionally, the high level of molecular complexity of a living cell makes testing difficult and reduces both specificity and reproducibility.

To overcome the limitations of detecting a ligand in a sample using cell-based assays, cell-free systems that detect a ligand in a sample where the ligand is capable of eliciting a steroid hormone genomic response have been developed.

However, a limitation of the cell-free assays is the requirement to use multi-subunit holoenzyme polymerases, such as RNA Polymerase II. The recombinant production of RNA polymerase II is extremely difficult to achieve with variable reproducibility, and as a consequence, the holoenzyme is typically made available by using nuclear extracts where all the components exist and come together at the promoter sequence. However, preparing nuclear extracts from eukaryotic cells is expensive to manufacture because of the need for costly cell growth media and the technical expertise required to enrich nuclear materials.

Advantageously, the present invention provides enzyme-free test kits, assays and methods which do not involve a transcription or translation event to detect the presence of a binding event, and therefore the presence of a ligand in a sample.

Lukman *et al.* (2013) describes a hybrid sensor using gold nanoparticles and conjugated polyelectrolytes for studying sequence rule in protein-DNA interactions.

Szatkowski Ozers *et al.* (1997) describes equilibrium binding of estrogen receptor with DNA using fluorescence anisotropy.

US 5945279 describes a screening system for identifying compounds that regulate steroid and orphan receptors mediation of DNA transcription.

### SUMMARY OF THE INVENTION

The invention provides a cell-free test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid reporter construct comprising:
   (a) a hormone response element that is capable of being bound by the receptor-ligand complex;
   (b) a fluorescence generating moiety; and
   (c) a fluorescence quenching moiety,
wherein the hormone response element (a) is located between the fluorescence generating moiety (b) and the fluorescence quenching moiety (c).

The invention also provides a cell-free assay method for detecting a ligand in a sample, which ligand is capable of eliciting a steroid hormone genomic response, the method comprising the steps of:
(i) contacting a sample with:
   (a) a steroid hormone receptor that forms a receptor-ligand complex with a ligand from the test sample;
   (b) optionally, heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52;
   (c) a nucleic acid reporter construct comprising:
      1. a hormone response element that is bound by the receptor-ligand complex;
      2. a fluorescence generating moiety; and
      3. a fluorescence quenching moiety,
   wherein the hormone response element (1) is located between the fluorescence generating moiety (2) and the fluorescence quenching moiety (3);
(ii) measuring a change in fluorescence of the reporter construct,
wherein, a measured change in the fluorescence of the reporter construct reflects that a ligand has been detected in the sample.

### FURTHER DISCLOSURE

In an aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid reporter construct comprising a hormone response element that is capable of being bound by the receptor-ligand complex;
wherein, the presence of a ligand in the sample is detected by measuring a change in a property of the reporter construct caused by binding of the receptor-ligand complex to the hormone response element when the sample is combined with the test kit.

In another aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid reporter construct comprising a hormone response element that is capable of being bound by the receptor-ligand complex; and
(iii) a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52,
wherein, the presence of a ligand in the sample is detected by measuring a change in a property of the reporter construct caused by binding of the receptor-ligand complex to the hormone response element when the sample is combined with the test kit.

In another aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid reporter construct comprising:
   (a) a hormone response element that is capable of being bound by the receptor-ligand complex; and
   (b) a fluorescence generating moiety,
wherein, the presence of a ligand in the sample is detected by measuring a change in the fluorescence of the reporter construct caused by binding of the receptor-ligand complex to the hormone response element when the sample is combined with the test kit.

In another aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid reporter construct comprising:
   (a) a hormone response element that is capable of being bound by the receptor-ligand complex; and
   (b) a fluorescence generating moiety; and
(iii) a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52,
wherein, the presence of a ligand in the sample is detected by measuring a change in the fluorescence of the reporter construct caused by binding of the receptor-ligand complex to the hormone response element when the sample is combined with the test kit.

In another aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid reporter construct comprising:
   (a) a hormone response element that is capable of being bound by the receptor-ligand complex; and
   (b) a fluorescence generating moiety,
wherein, the presence of a ligand in the sample is detected by measuring a decrease in the fluorescence of the reporter construct caused by binding of the receptor-ligand complex to the hormone response element when the sample is combined with the test kit.

In another aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid reporter construct comprising:
   (a) a hormone response element that is capable of being bound by the receptor-ligand complex; and
   (b) a fluorescence generating moiety,
(iii) a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52,
wherein, the presence of a ligand in the sample is detected by measuring a decrease in the fluorescence of the reporter construct caused by binding of the receptor-ligand complex to the hormone response element when the sample is combined with the test kit.

In another aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid reporter construct comprising:
   (a) a hormone response element that is capable of being bound by the receptor-ligand complex; and
   (b) a fluorescence generating moiety,
wherein, the presence of a ligand in the sample is detected by measuring an increase in the fluorescence of the reporter construct caused by binding of the receptor-ligand complex to the hormone response element when the sample is combined with the test kit.

In another aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid reporter construct comprising:
   (a) a hormone response element that is capable of being bound by the receptor-ligand complex; and
   (b) a fluorescence generating moiety,
(iii) a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52,
wherein, the presence of a ligand in the sample is detected by measuring an increase in the fluorescence of the reporter construct caused by binding of the receptor-ligand complex to the hormone response element when the sample is combined with the test kit.

In yet another aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid reporter construct comprising:
   (a) a hormone response element that is capable of being bound by the receptor-ligand complex;
   (b) a fluorescence generating moiety; and
   (c) a fluorescence quenching moiety,
wherein, the presence of a ligand in the sample is detected by measuring a change in fluorescence of the reporter construct caused by binding of the receptor-ligand complex to the hormone response element when the sample is combined with the test kit.

In yet another aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid reporter construct comprising:
   (a) a hormone response element that is capable of being bound by the receptor-ligand complex;
   (b) a fluorescence generating moiety; and
   (c) a fluorescence quenching moiety,
(iii) a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52,
wherein, the presence of a ligand in the sample is detected by measuring a change in fluorescence of the reporter construct caused by binding of the receptor-ligand complex to the hormone response element when the sample is combined with the test kit.

In yet another aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid reporter construct comprising:
   (a) a hormone response element that is capable of being bound by the receptor-ligand complex;
   (b) a fluorescence generating moiety comprising a quantun dot (QD); and
   (c) a fluorescence quenching moiety comprising a gold nanoparticle,
wherein, the presence of a ligand in the sample is detected by measuring a change in fluorescence of the reporter construct caused by binding of the receptor-ligand complex to the hormone response element when the sample is combined with the test kit.

In yet another aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid reporter construct comprising:
   (a) a hormone response element that is capable of being bound by the receptor-ligand complex;
   (b) a fluorescence generating moiety comprising a quantun dot (QD); and
   (c) a fluorescence quenching moiety comprising a gold nanoparticle,
(iii) a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52,
wherein, the presence of a ligand in the sample is detected by measuring a change in fluorescence of the reporter construct caused by binding of the receptor-ligand complex to the hormone response element when the sample is combined with the test kit.

In yet another aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid reporter construct comprising:
   (a) a hormone response element that is capable of being bound by the receptor-ligand complex;
   (b) a fluorescence generating moiety; and
   (c) a fluorescence quenching moiety,
wherein, the presence of a ligand in the sample is detected by measuring an increase in fluorescence of the reporter construct caused by binding of the receptor-ligand complex to the hormone response element when the sample is combined with the test kit.

In yet another aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid reporter construct comprising:
   (a) a hormone response element that is capable of being bound by the receptor-ligand complex;
   (b) a fluorescence generating moiety; and
   (c) a fluorescence quenching moiety,
(iii) a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52,
wherein, the presence of a ligand in the sample is detected by measuring an increase in fluorescence of the reporter construct caused by binding of the receptor-ligand complex to the hormone response element when the sample is combined with the test kit.

In yet another aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid reporter construct comprising:
   (a) a hormone response element that is capable of being bound by the receptor-ligand complex;
   (b) a fluorescence generating moiety comprising a quantum dot; and
   (c) a fluorescence quenching moiety comprising a gold nanoparticle,
wherein, the presence of a ligand in the sample is detected by measuring an increase in fluorescence of the reporter construct caused by binding of the receptor-ligand complex to the hormone response element when the sample is combined with the test kit.

In yet another aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid reporter construct comprising:
   (a) a hormone response element that is capable of being bound by the receptor-ligand complex;
   (b) a fluorescence generating moiety comprising a quantum dot; and
   (c) a fluorescence quenching moiety comprising a gold nanoparticle,
(iii) a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52,
wherein, the presence of a ligand in the sample is detected by measuring an increase in fluorescence of the reporter construct caused by binding of the receptor-ligand complex to the hormone response element when the sample is combined with the test kit.

In yet another aspect of the present disclosure there is described an assay method for detecting a ligand in a sample which ligand is capable of eliciting a steroid hormone genomic response, the method comprising the steps of:
(i) contacting a sample with a test kit as described herein; and
(ii) measuring a change in a physical property of the reporter construct caused by binding of the receptor-ligand complex to the hormone response element,
wherein, a measured change in a physical property of the reporter construct reflects detection of a ligand in the sample.

In yet another aspect of the present disclosure there is described an assay method for detecting a ligand in a sample which ligand is capable of eliciting a steroid hormone genomic response, the method comprising the steps of:
(i) contacting a sample with a test kit comprising a fluorescence-based reporter construct as described herein; and
(ii) measuring a change in fluorescence of the reporter construct caused by binding of the receptor-ligand complex to the response element,
wherein, a measured change in fluorescence of the reporter construct reflects detection of a ligand in the sample.

In yet another aspect of the present disclosure there is described an assay method for detecting a ligand in a sample which ligand is capable of eliciting a steroid hormone genomic response, the method comprising the steps of:
(i) contacting a sample with a test kit comprising a fluorescence-based reporter construct as described herein; and
(ii) measuring a change in fluorescence of the reporter construct caused by binding of the receptor-ligand complex to the hormone response element,
wherein, a measured change in fluorescence of the reporter construct reflects detection of a ligand in the sample.

In yet another aspect of the present disclosure there is described an assay method for detecting a ligand in a sample which ligand is capable of eliciting a steroid hormone genomic response, the method comprising the steps of:
(i) contacting a sample with a test kit comprising a fluorescence-based reporter construct as described herein; and
(ii) measuring an increase in fluorescence of the reporter construct caused by binding of the receptor-ligand complex to the hormone response element,
wherein, a measured increase in fluorescence of the reporter construct reflects detection of a ligand in the sample.

In a further aspect of the present disclosure there is described a method for determining the steroid hormone bioactivity of a sample, the method comprising combining a sample with a test kit as described herein to ascertain if the sample comprises a ligand sufficient to activate a steroid hormone receptor and cause a change in a physical property of the reporter construct, wherein a change in a physical property of the reporter construct provides information about the steroid hormone bioactivity of the sample.

In a further aspect of the present disclosure there is described a method for determining the steroid hormone bioactivity of a biological sample, the method comprising combining a biological sample with a test kit as described herein to ascertain if the sample comprises a ligand sufficient to activate a steroid hormone receptor and cause a change in a physical property of the reporter construct, wherein a change in a physical property of the reporter construct provides information about the steroid hormone bioactivity of the biological sample.

In a further aspect of the present disclosure there is described a method for determining the steroid hormone bioactivity of a clinical specimen, the method comprising combining a sample obtained from the clinical specimen with a test kit as described herein to ascertain if the sample comprises a ligand sufficient to activate a steroid hormone receptor and cause a change in a physical property of the reporter construct, wherein a change in a physical property of the reporter construct provides information about the steroid hormone bioactivity of the clinical specimen.

In a further aspect of the present disclosure there is described a method for determining the steroid hormone bioactivity of a food or a nutritional supplement, the method comprising combining the food or nutritional supplement, or an extract of the food or nutritional supplement, with a test kit as described herein to ascertain if the sample comprises a ligand sufficient to activate a steroid hormone receptor and cause a change in a physical property of the reporter construct, wherein a change in a physical property of the reporter construct provides information about the steroid hormone bioactivity of the food or nutritional supplement.

In a further aspect of the present disclosure there is described a method for determining the steroid hormone bioactivity of a sample derived from an environmental source, the method comprising combining a sample obtained from an environmental source with a test kit as described herein to ascertain if the sample comprises a ligand sufficient to activate a steroid hormone receptor and cause a change in a physical property of the reporter construct, wherein a change in a physical property of the reporter construct provides information about the steroid hormone bioactivity of the environmental sample.

In a further aspect of the present disclosure there is describeda method for determining the doping status of an athlete, the method comprising combining a sample obtained from an athlete with a test kit as described herein to ascertain if the sample comprises a ligand sufficient to activate a steroid hormone receptor and cause a change in a physical property of the reporter construct, wherein a change in a physical property of the reporter construct provides information about the doping status of the athlete.

In a further aspect of the present disclosure there is described a method for determining the steroid hormone bioactivity of a sample, the method comprising performing an assay method as described herein on a sample to ascertain if the sample comprises a ligand sufficient to activate a steroid hormone receptor and cause a change in a physical property of the reporter construct, wherein a change in a physical property of the reporter construct provides information about the steroid hormone bioactivity of the sample.

In a further aspect of the present disclosure there is described a method for determining the steroid hormone bioactivity of a biological sample, the method comprising performing an assay method as described herein on a sample to ascertain if the sample comprises a ligand sufficient to activate a steroid hormone receptor and cause a change in a physical property of the reporter construct, wherein a change in a physical property of the reporter construct provides information about the steroid hormone bioactivity of the biological sample.

In a further aspect of the present disclosure there is described a method for determining the steroid hormone bioactivity of a food or a nutritional supplement, the method comprising performing an assay method as described herein on the food or nutritional supplement, or an extract from the food or nutritional supplement, to ascertain if the food or nutritional supplement comprises a ligand sufficient to activate a steroid hormone receptor and cause a change in a physical property of the reporter construct, wherein a change in a physical property of the reporter construct provides information about the steroid hormone bioactivity of the food or nutritional supplement.

In a further aspect of the present disclosure there is described a method for determining the steroid hormone bioactivity of a clinical specimen, the method comprising performing an assay method as described herein on a clinical specimen to ascertain if the sample comprises a ligand sufficient to activate a steroid hormone receptor and cause a change in a physical property of the reporter construct, wherein a change in a physical property of the reporter construct provides information about the steroid hormone bioactivity of the clinical specimen.

In a further aspect of the present disclosure there is described a method for determining the steroid hormone bioactivity of a sample derived from an environmental source, the method comprising performing an assay method as described herein on the environmental sample to ascertain if the sample comprises a ligand sufficient to activate a steroid hormone receptor and cause a change in a physical property of the reporter construct, wherein a change in a physical property of the reporter construct provides information about the steroid hormone bioactivity.

In a further aspect of the present disclosure there is described a method for determining the doping status of an athlete, the method comprising performing an assay method as described herein on a sample obtained from the athlete to ascertain if the sample comprises a ligand sufficient to activate a steroid hormone receptor and cause a change in a physical property of the reporter construct, wherein a change in a physical property of the reporter construct provides information about the doping status of the athlete.

In a further aspect of the present disclosure there is described an article of manufacture for screening a sample for the presence of a ligand, which ligand is capable of eliciting a steroid hormone genomic response, the article of manufacture comprising a test kit as described herein together with instructions for how to detect the presence of a ligand in the sample.

In a further aspect of the present disclosure there is described an article of manufacture for screening a biological sample for the presence of a ligand, which ligand is capable of eliciting a steroid hormone genomic response, the article of manufacture comprising a test kit as described herein together with instructions for how to detect the presence of a ligand in the biological sample.

In a further aspect of the present disclosure there is described an article of manufacture for screening a food or a nutritional supplement for the presence of a ligand, which ligand is capable of eliciting a steroid hormone genomic response, the article of manufacture comprising a test kit as described herein together with instructions for how to detect the presence of a ligand in the food or nutritional supplement.

In yet a further aspect of the present disclosure there is described an article of manufacture for determining the steroid hormone bioactivity of a sample, the article of manufacture comprising a test kit as described herein together with instructions for detecting the steroid hormone bioactivity in a sample, wherein the presence of bioactive ligands in the sample is indicative of steroid hormone bioactivity of the sample.

In yet a further aspect of the present disclosure there is described an article of manufacture for determining the steroid hormone bioactivity of a clinical specimen, the article of manufacture comprising a test kit as described herein together with instructions for detecting the steroid hormone bioactivity in a clinical specimen, wherein the presence of bioactive ligands in the clinical specimen is indicative of steroid hormone bioactivity of the clinical specimen.

In yet a further aspect of the present disclosure there is described an article of manufacture for determining doping in an athlete, the article of manufacture comprising a test kit as described herein together with instructions for detecting the presence of a ligand in a sample derived from the athlete, wherein the presence of the ligand in the sample is indicative of doping in the athlete.

### BRIEF DESCRIPTION OF THE FIGURES

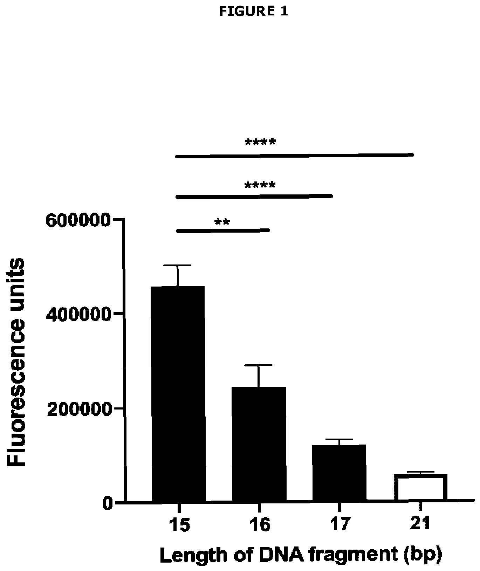

**Figure 1** **shows** that the length of DNA fragment directly impacts FRET output. Cy3-labelled sense strands were annealed to Cy5-labelled antisense strands. The double-stranded DNA fragment was annealed in in reaction buffer (20mM HEPES pH 7.9, 100mM KCl, 20% glycerol). A steroid response element is 15bp in length. A 15 bp DNA fragment encoding androgen response element (ARE) was shown to be of suitable length to allow FRET to take place between Cy3 and Cy5. FRET between the Cy3 and Cy5 was determined using a fluorescence plate reader, with 540 nm excitation and 680m emission. Longer DNA fragments to allow inclusion of additional base pairs to enhance protein-DNA interaction were tried. These data show that as the length of DNA increases, the detection of FRET between Cy3 and Cy5 decreases. P=0.008 for 15bp vs 16bp, and P<0.001 for 15bp vs 17 bp or 21 bp.

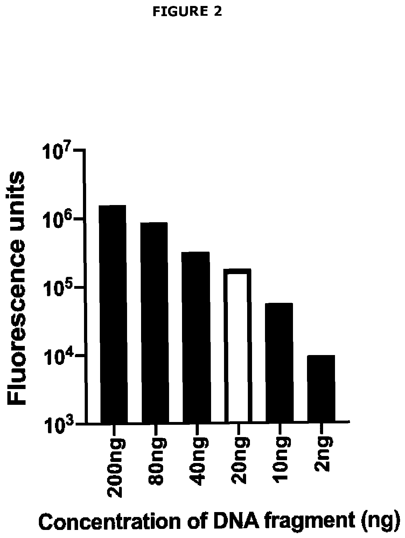

**Figure 2** **shows** that concentration of DNA fragment directly impacts FRET output. The data shows that a titration of DNA fragment from 200ng to 2ng results in a two orders of magnitude difference in fluorescence output. The result of decreased fluorescence is in keeping with the two orders of magnitude difference in starting DNA concentration.

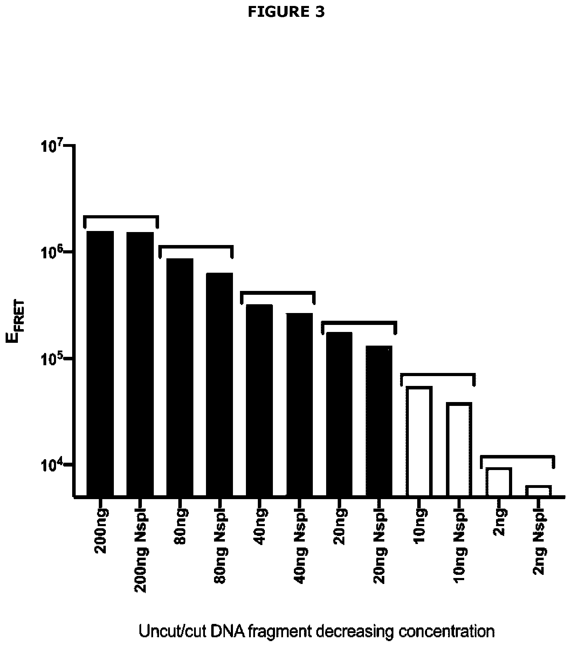

**Figure 3** **shows** that concentration of DNA fragment directly impacts the ability to detect change in FRET output (ΔE_{FRET}). The data shows the uncut versus cut DNA fragment for each concentration. It was not until the DNA concentration was at 2-10ng that easily detectable differences in (ΔE_{FRET}) could be measured.

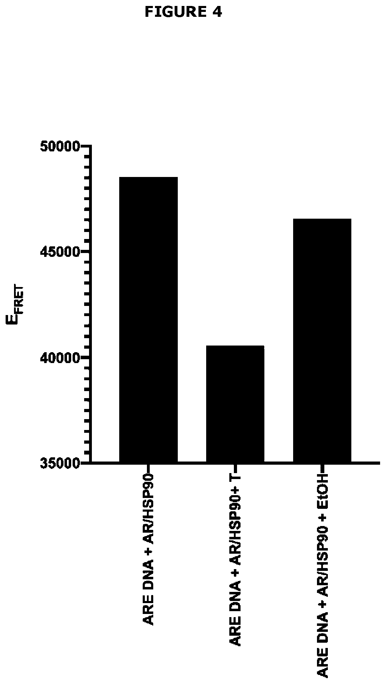

**Figure 4** **shows** that ΔE_{FRET} measured for testosterone-activated AR reaction with 10ng ARE DNA concentration. Reactions were assembled as described in the text. Data shows that AR activated by testosterone (T) shows a decrease in E_{FRET} that was not measured in either control reaction. ARE DNA is the DNA fragment (10ng), AR is the androgen receptor, HSP90 is the inhibitor protein, T is 250 µM, and EtOH is ethanol, the hydrophobic diluent used to dissolve and deliver testosterone.

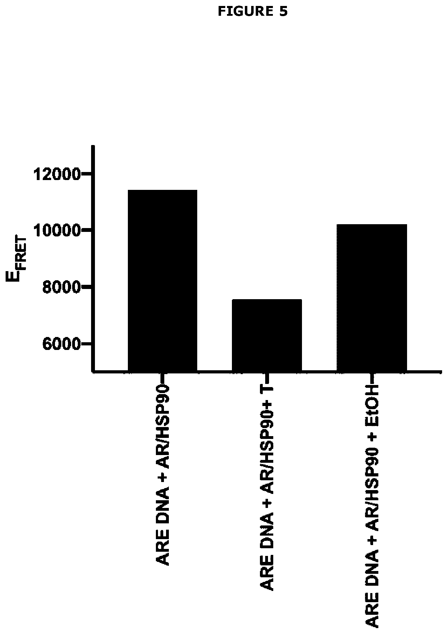

**Figure 5** **shows** that ΔE_{FRET} measured for testosterone-activated AR reaction with 2ng ARE DNA concentration. Reactions were assembled as described in the text. Data shows that AR activated by testosterone (T) shows a decrease in E_{FRET} that was not measured in either control reaction. ARE DNA is the DNA fragment (2 ng), AR is the androgen receptor, HSP90 is the inhibitor protein, T is 250 µM final concentration, and EtOH is ethanol, the hydrophobic diluent used to dissolve and deliver testosterone.

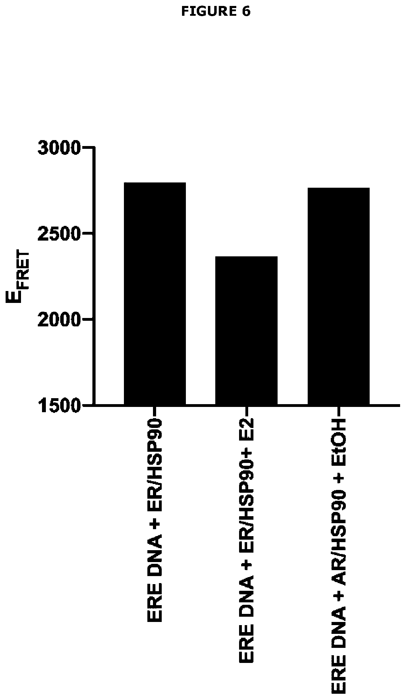

**Figure 6** **shows** that ΔE_{FRET} measured for estradiol-activated ERa reaction with 2ng ERE DNA concentration. Reactions were assembled as described in the text. Data shows that ER_{α} activated by estradiol (E2) shows a decrease in E_{FRET} that was not measured in either control reaction. ERE DNA is the DNA fragment (2 ng), ERa is the estrogen receptor, HSP90 is the inhibitor protein, E2 is 5 nM final concentration, and EtOH is ethanol, the hydrophobic diluent used to dissolve and deliver estradiol.

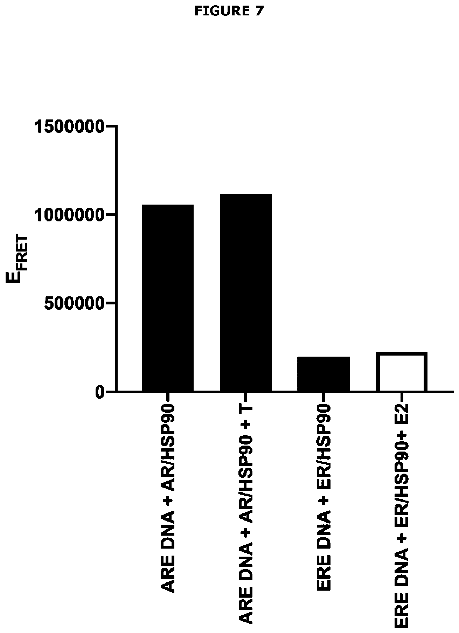

**Figure 7** **shows** that ΔE_{FRET} measured for testosterone-activated AR reaction or estradiol-activated ERa reaction with 40ng DNA fragment concentration. Reactions were assembled as described in the text. Data shows that ligand-activated AR or ER_{α} did not induce a decrease in E_{FRET} when the DNA fragment concentration was 40 ng.

### GENERAL DEFINITIONS

Unless specifically defined otherwise, all technical and scientific terms used herein shall be taken to have the same meaning as commonly understood by one of ordinary skill in the art (for example, in immunology, immunohistochemistry, protein chemistry, molecular genetics, synthetic biology and biochemistry).

It is intended that reference to a range of numbers disclosed herein (e.g. 1 to 10) also incorporates all related numbers within that range (e.g. 1, 1.1, 2, 3, 3.9, 4, 5, 6, 6.5, 7, 8, 9 and 10) and also any range of rational numbers within that range (for example 2 to 8, 1.5 to 5.5 and 3.1 to 4.7) and, therefore, all sub-ranges of all ranges expressly disclosed herein are expressly disclosed. These are only examples of what is specifically intended and all possible combinations of numerical values between the lowest value and the highest value enumerated are to be considered to be expressly stated in this application in a similar manner.

The term "and/or", e.g., "X and/or Y" shall be understood to mean either "X and Y" or "X or Y" and shall be taken to provide explicit support for both meanings or for either meaning.

The term "a" or "an" refers to one or more than one of the entity specified; for example, "a receptor" or "a nucleic acid molecule" may refer to one or more receptor or nucleic acid molecule, or at least one receptor or nucleic acid molecule. As such, the terms "a" or "an", "one or more" and "at least one" can be used interchangeably herein.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

Throughout this specification, unless specifically stated otherwise or the context requires otherwise, reference to a single step, composition of matter, group of steps or group of compositions of matter shall be taken to encompass one and a plurality (i.e. one or more) of those steps, compositions of matter, groups of steps or group of compositions of matter.

### SELECTED DEFINITIONS

For the purposes of the present disclosure, the following terms shall have the following meanings.

The term "test kit" as used herein refers to an article of manufacture comprising various components to perform the assays and methods described herein.

The term "steroid hormone receptor" or "SHR" as used herein refers to a protein or polypeptide, including recombinant polypeptides that selectively binds to a ligand, which ligand is capable of activating the steroid hormone receptor, and includes, without limitation, an androgen receptor, an estrogen receptor, a progesterone receptor, a mineralocorticoid receptor and a glucocorticoid receptor. Typically, a steroid hormone receptor comprises a ligand binding domain, an activation domain and a deoxyribose nucleic acid binding domain. According to this definition, "steroid hormone receptor" may optionally include other cofactors, including (e.g.) heat shock proteins and the like, which help to hold the steroid hormone receptor in a folded and hormone responsive state for activation by a ligand.

The term "steroid hormone receptor cofactor" as used herein refers to one or more cofactors that hold the steroid hormone receptor in an inactive state, until displaced by a ligand, at which point the steroid hormone receptor becomes activated. Examples of steroid hormone receptor cofactors include, without limitation, heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52.

The term "ligand" refers generally to any molecule that binds to a receptor, and includes without limitation, a steroid, a polypeptide, a protein, a vitamin, a carbohydrate, a glycoprotein, a therapeutic agent, a drug, a glycosaminoglycan, or any combination thereof. As used herein, "ligand" includes, without limitation, steroid hormones, such as sex hormones including but not limited to estrogens, progestagens, androgens etc, as well as natural and synthetic derivatives and analogs and metabolites thereof, designer steroid hormones, anabolic androgenic steroids, and selective androgen-, progestagen- and estrogen receptor modulators, those that are currently known and those anticipated to be developed or naturally found in biological samples.

The term "receptor-ligand complex" and "activated steroid hormone receptor" as used interchangeably herein to refer to a ligand bound steroid hormone receptor, where the steroid hormone receptor undergoes a structural transformation upon binding the ligand and is then said to be in an activated form. A receptor-ligand complex as described herein includes, without limitation, a dimer of a ligand bound hormone receptor (i.e. (HR-L)₂.

The term "steroid metabolism machinery" as used herein refers to any enzyme, and includes combinations of enzyme, sufficient to convert a ligand from a physiologically inactive form to a physiologically active form or from a physiologically active form to a *more* physiologically active form, or from a physiologically active form to a less physiologically active form, or from a physiologically active form to a physiologically inactive form.

The term "detection means" as used herein refers to any apparatus, equipment or configuration adapted to detect the binding interaction between an activated steroid hormone receptor and nucleic acid response element. Examples of detection means include, but are not limited to, optical methods, spectroscopy, visible spectroscopy, Raman spectroscopy, UV spectroscopy, surface plasmon resonance, electrochemical methods, impedance, resistance, capacitance, mechanical sensing by changes in mass, changes in mechanical resonance, electrophoresis, gel electrophoresis, gel retardation, imaging, fluorescence and fluorescence resonance energy transfer, polymerase chain reaction *etc.*

The term "nucleic acid sequence" as used herein refers to a deoxyribose nucleic acid (DNA) sequence, a ribose nucleic acid sequence (RNA), messenger ribose nucleic acid (mRNA) and complementary DNA (cDNA), and is comprised of a continuous sequence of two or more nucleotides, also referred to as a polynucleotide or oligonucleotide. The nucleic acid sequence may be single-stranded or double-stranded.

The term "sample" as used herein refers to any sample for which it is desired to test for the presence of a ligand. The terms "sample" and "test sample" are used interchangeably in this specification.

The term "relative potency" or "RP" as used herein refers to the multiplier of biological activity exhibited by a test compound relative to a reference compound, where the biological activity is defined by the ability of the compound to bind to and activate a steroid hormone receptor (e.g.) as measured using the assays and test kits described herein. Where Relative Potency is > 1, the test compound is more potent in terms of its biological activity as compared to the reference compound; where Relative Potency is < 1, the test compound is less potent in terms of its biological activity as compared to the reference compound; and where Relative Potency =1, the test and reference compounds are equally potent in terms of their biological activities.

The term "activation factor" or "AF" as used herein relates to the measure of metabolic conversion of a test compound (e.g.) from a physiologically inactive state to a physiologically active state or from a less physiologically active state to a more physiologically active state. An activation factor >1 means that the test compound has undergone metabolic conversion to a more physiologically active state in the presence of metabolic machinery in the assay.

The term "reference threshold" or "reference standard" may be used interchangeably to means the level of assay activity measured (e.g.) in the absence of a test sample, or in the absence of test sample and steroid hormone receptor. In certain examplesdescribed herein, the reference threshold or reference standard is determined using ethanol in place of test sample. The reference threshold is intended to establish any baseline activity or signal of the assay in the absence of target ligand.

### DETAILED DESCRIPTION

The invention is defined in the independent claims. Further aspects and preferred embodiments are defined in the dependent claims. Any aspects, embodiments and examples of the present disclosure which do not fall under the scope of the appended claims do not form part of the invention and are merely provided for illustrative purposes.

The present disclosure describes test kits, assays and methods useful for screening a sample for the presence of a ligand capable of activating a steroid hormone receptor and eliciting a steroid hormone genomic response.

In certain examples according to the present disclosure, the test kits, assays and methods described herein are useful for determining the hormone status of a subject, for example, by measuring the androgenic and/or estrogenic activity of a sample obtained from the subject. This information may then be used to determine, for example, whether the subject has, or is at risk for developing, cancer, or for investigating endocrine issues associated with ageing such as, for example, menopause or for evaluating the efficacy of hormone replacement therapy or hormone inhibitory therapy.

In other examples according to the present disclosure, the test kits, assays and methods described herein are useful for screening foods and health food supplements for banned additives or for natural or synthetic activators that could be harmful to health, including, but not limited to, phyto- or xeno-estrogens that could promote hormone sensitive cancers.

### Non-Enzyme Mediated Activity Assays & Test Kits

The assays according to the present disclosure, on which the test kits and methods described herein are based, are fundamentally activity/function based assays which work on the principle of steroid hormone receptor activation through binding of a ligand derived from a sample to be tested. Activation of a steroid hormone receptor occurs when a ligand binds to the receptor and induces a conformational change in the tertiary structure of the protein, meaning that the receptor-ligand complex (also referred to herein as an *'*activated steroid hormone receptor') is then able to bind to a nucleic acid response element and elicit a so-called 'genomic response'. In other words the ability to up- or down-regulate expression of genes from the genome in the nucleus of the cell which may then lead to a physiological effect. It is this binding interaction between the activated steroid hormone receptor and nucleic acid response element that is measured by the test kits, assays and methods described herein, as a proxy to detect the presence of a ligand having *steroid-, or steroid-like* activity in a sample under investigation.

Importantly, this means the test kits and assays according to the present disclosure have the ability to detect steriod hormone bio/activity elicited by ligands of unknown structure such as (e.g.) *'*designer drugs*'*. Historically, this has not been possible since conventional laboratory testing equipment, such as gas/liquid chromatography and mass spectrometry, requires prior knowledge of the structure of the molecule being investigated.

Previous approaches to detection of steroid hormone bio/activity has involved yeast and mammalian cell reporter assays (e.g. refer to Cooper et al. (2013) Sensors 13:2148-2163). Such assays have well-recognised limitations such as the need for (e.g.) specialised equipment and expertise to maintain living cell cultures. The development of cell-free assays modeled on molecular frameworks that largely mimic cell-based systems has generated *in vitro* assays with enhanced funtionality and improved assay sensitivity compared to its cell-based reporter counterpart(s). However, a limitation of the cell-free assays is the requirement to use multi-subunit holoenzyme polymerases, such as RNA Polymerase II. The recombinant production of RNA polymerase II is extremely difficult to achieve with variable reproducibility, and as a consequence, the holoenzyme is typically made available by using nuclear extracts where all the components exist and come together at the promoter sequence. However, preparing nuclear extracts from eukaryotic cells is expensive to manufacture because of the need for costly cell growth media and the technical expertise required to enrich nuclear materials.

To address this limitation, Applicants have now developed cell-free and enzyme-free assays which function by measuring a change in a property of a reporter construct caused by the presence of a ligand. For example, an increase, decrease or inhibition, or activation of signal generated by the reporter construct.

In an aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid reporter construct comprising a hormone response element that is capable of being bound by the receptor-ligand complex;
wherein, the presence of a ligand in the sample is detected by measuring a change in a property of the reporter construct caused by binding of the receptor-ligand complex to the hormone response element when the sample is combined with the test kit.

In yet another aspect of the present disclosure there is described an assay method for detecting a ligand in a sample which ligand is capable of eliciting a steroid hormone genomic response, the method comprising the steps of:
(i) contacting a sample with a test kit as described herein; and
(ii) measuring a change in a physical property of the reporter construct caused by binding of the receptor-ligand complex to the hormone response element,
wherein, a measured change in a physical property of the reporter construct reflects detection of a ligand in the sample.

In an example according to the test kits and assay methods described herein, the presence of a ligand in the test sample is detected by measuring a change in a physical property of the reporter construct.

In another example according to the test kits and assay methods described herein, the presence of a ligand in the test sample is detected by measuring a change in a mechanical property of the reporter construct.

In another example according to the test kits and assay methods described herein, the presence of a ligand in the test sample is detected by measuring a change in an optical property of the reporter construct.

In another example according to the test kits and assay methods described herein, the presence of a ligand in the test sample is detected by measuring a change in a photochemical property of the reporter construct.

In another example according to the test kits and assay methods described herein, the presence of a ligand in the test sample is detected by measuring a change in an electrochemical property of the reporter construct.

In another example according to the test kits and assay methods described herein, the presence of a ligand in the test sample is detected by measuring a change in fluorescence associated with the reporter construct, and wherein the reporter construct comprises fluorescence generating moieties.

In yet another example according to the test kits and assay methods described herein, binding of the receptor-ligand complex to the nucleic acid reporter construct is measured using methods including, but not limited to, optical methods, spectroscopy, visible spectroscopy, Raman spectroscopy, UV spectroscopy, surface plasmon resonance, electrochemical methods, impedance, resistance, capacitance, mechanical sensing by changes in mass, changes in mechanical resonance, electrophoresis, gel electrophoresis, gel retardation, imaging, fluorescence, and fluorescence resonance energy transfer.

A difficulty in leveraging molecular frameworks derived from cellular systems is the complexity associated with the way in which cellular systems are fine-tuned to optimise signalling events. To illustrate this point, a steroid hormone receptor may take on different tertiary structure conformations which impart different biological functions. For example, ligand binding to a steroid hormome receptor induces a conformational change in the tertiary structure of the receptor protein, which enables it to form a dimer with another 'activated' steriod hormone receptor-ligand molecule, which dimer binds to a nucleic acid response element. A second pathway is known where, in the nucleus, a single 'activated' steriod hormone receptor-ligand binds to a nucleic acid response element followed by the binding of second 'activated' steriod hormone receptor-ligand to the same nucleic acid response element. Both pathways within the nucleus causes up- or down-regulation of gene expression referred to herein as a steroid hormone receptor genomic response.

In the cellular environment, the non-ligand bound steriod hormone receptor is held in an inactive conformation by chaperone proteins including but not limited to, heat shock proteins. In this inactive conformation, little or no activation of the corresponding response element is possible in the absence of ligand because the DNA binding domain is not exposed and also because for a some steroid hormone receptors they are located in the cytoplasm and have not been targeted for translocation to the nucleus.

Accordingly, in a further example the test kits and assay methods described herein further comprises at least one steroid hormone receptor cofactor that holds the steroid hormone receptor in an inactive state (i.e. for ligand binding) and which significantly diminishes or inhibits its ability to bind to the hormone response element in the absence of a ligand.

In a related example, the steroid hormone receptor cofactor is selected from one or more of heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52,.

In another example according to the present disclosure, the test kits and assay methods further comprise heat shock protein 90.

In a further aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid reporter construct comprising a hormone response element that is capable of being bound by the receptor-ligand complex; and
(iii) a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52,
wherein, the presence of a ligand in the sample is detected by measuring a change in a property of the reporter construct caused by binding of the receptor-ligand complex to the hormone response element when the sample is combined with the test kit.

In yet another aspect of the present disclosure there is described an assay method for detecting a ligand in a sample which ligand is capable of eliciting a steroid hormone genomic response, the method comprising the steps of:
(i) contacting a sample with a test kit as described herein; and
(ii) measuring a change in a physical property of the reporter construct caused by binding of the receptor-ligand complex to the hormone response element,
wherein, a measured change in a physical property of the reporter construct reflects detection of a ligand in the sample.

It should, however, be appreciated that the presence of steroid hormone receptor cofactor is not an essential feature of the test kits and assay methods described herein because an assay result may still be achieved in the absence of cofactor.

Indeed, Applicants have observed a differential in the binding affinty/kinetics between ligand bound and non-ligand bound steroid hormone receptor for the hormone response element. Accordingly, test kits and assay methods described herein may be performed at a temperature, or in a temperature range, that preferentially measures ligand-bound receptor over non-ligand bound receptor, thereby minimising any background signal generated by non-ligand bound receptor.

Accordingly, in yet another example according to this aspect of the present disclosure, performance of the test kits and assay methods described herein is in a temperature range from about 25 °C to about 42 °C, preferably about 35 to about 37 °C.

The term "a temperature range from about 25 °C to about 42 °C" is intended to include any temperature from 25 °C to 42 °C and without limitation includes 25 °C, 26 °C, 27 °C, 28 °C, 29 °C, 30 °C, 31 °C, 32 °C, 33 °C, 34 °C, 35 °C, 36 °C, 37 °C, 38 °C, 39 °C, 40 °C, 41 °C and 42 °C. The skilled person would recognise that temeratures in the decimal point range may also be used. To further illustrate this point, "in a temperature range from about 35 °C to about 37 °C" includes, without limitation, 35.0 °C, 35.1 °C, 35.2 °C, 35.3 °C, 35.4 °C, 35.5 °C, 35.6 °C, 35.7 °C, 35.8 °C, 35.9 °C, 36.0 °C, 36.1 °C, 36.2 °C, 36.3 °C, 36.4 °C, 36.5 °C, 36.6 °C, 36.7 °C, 36.8 °C, 36.9 °C and 37.0 °C.

In yet another example according to the test kits and assay methods described herein, a modified form of the steroid hormone receptor with decreased affinity for the hormone response element in the the absence of bound ligand is employed.

An approach to detection of a ligand in a sample would be to use a fluorescence based reporter construct, and to measure a change in the fluorescence properties of the reporter construct caused when a receptor-ligand complex binds to the hormone response element located within the reporter construct.

There are various configurations envisaged. For example, nucleic acid reporter construct inclusive of a hormone response element is modified to include a fluorescence generating molecule (e.g.) fluorophore that can be modulated by the proximity of at least one additional fluorophore or fluorescence quenching molecule to enable static quenching, Förster Resonance Energy Transfer (FRET) quenching or a combination of both.

Static quenching occurs when molecules form a complex in the ground state, i.e. before excitation occurs, whereas Förster Resonance Energy Transfer is a dynamic quenching mechanism because energy transfer occurs while the donor is in the excited state.

When a receptor-ligand complex binds to the hormone response element, it forces a change in the spatial relationship between (e.g.) donor and acceptor fluorophores, thereby altering the level of static quenching or FRET quenching or a combination of both. The result is an increase or decrease in the amount of measurable fluorescence, reflecting the presence of a target ligand in a sample under investigation.

Accordingly, in another aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid reporter construct comprising:
   (a) a hormone response element that is capable of being bound by the receptor-ligand complex; and
   (b) a fluorescence generating moiety,
wherein, the presence of a ligand in the sample is detected by measuring a change in fluorescence signal of the reporter construct caused by binding of the receptor-ligand complex to the hormone response element when the sample is combined with the test kit.

In another aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid reporter construct comprising:
   (a) a hormone response element that is capable of being bound by the receptor-ligand complex; and
   (b) a fluorescence generating moiety; and
(iii) a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52,
wherein, the presence of a ligand in the sample is detected by measuring a change in the fluorescence of the reporter construct caused by binding of the receptor-ligand complex to the hormone response element when the sample is combined with the test kit.

The present disclosure further contemplates assay methods on which performance of the test kits described herein are based.

Accordingly, in yet another aspect of the present disclosure there is described an assay method for detecting a ligand in a sample, which ligand is capable of eliciting a steroid hormone genomic response, the method comprising the steps of:
(i) contacting a test sample with:
   (a) a steroid hormone receptor that forms a receptor-ligand complex with a ligand from the test sample; and
   (b) a nucleic acid reporter construct comprising:
      i. a hormone response element that is bound by the receptor-ligand complex; and
      ii. a fluorescence generating moiety; and
(ii) measuring the fluorescence signal of the reporter construct,
wherein, a measured change in fluorescence of the reporter construct reflects that a ligand has been detected in the sample.

In yet a further aspect of the present disclosure there is described an assay method for detecting a ligand in a sample, which ligand is capable of eliciting a steroid hormone genomic response, the method comprising the steps of:
(i) contacting a test sample with:
   (a) a steroid hormone receptor that forms a receptor-ligand complex with a ligand from the test sample; and
   (b) a nucleic acid reporter construct comprising:
      1. a hormone response element that is bound by the receptor-ligand complex; and
      2. a fluorescence generating moiety; and
   (c) a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52,
(ii) measuring the fluorescence signal of the reporter construct,
wherein, a measured change in fluorescence of the reporter construct reflects that a ligand has been detected in the sample.

In an example according to the test kits and assay methods involving fluorescence based reporter constructs, the fluorescence generating moiety comprises a donor molecule and an acceptor molecule.

In an example, the donor molecule comprises a fluorophore.

In another example, the donor molecule comprises a quantum dot.

In an example, the acceptor molecule comprises a fluorophore.

In another example, the acceptor molecule comprises a gold nanoparticle.

In a related example, the donor molecule comprises a quantum dot and the acceptor molecule comprises a gold nanoparticle.

In a related example, the donor and acceptor molecules are held in a structural conformation that allows energy transfer between the donor molecule and the acceptor molecule in the absence of sample comprising a target ligand.

In a related example, the structural conformation includes, without limitation, linear, circular and U-shaped conformations.

In a related example, the reporter construct generates a measurable fluorescence signal in the absence of sample.

In a related example, a delta in the level or amount of fluorescence reflects the presence of a target ligand in the sample during performance of the test kit or assay method.

According to the Prototype Assays described in the Examples which follow, Applicants developed constructs which employ Förster Resonance Energy Transfer because it is very sensitive to nanoscale changes in distance between molecules. Förster Resonance Energy Transfer is a nonradiative process whereby an excited state donor (usually a fluorophore) transfers energy to a proximal ground state acceptor through long-range dipole-dipole interactions. The rate of energy transfer is highly dependent on many factors, such as the extent of spectral overlap, the relative orientation of the transition dipoles, and, most importantly, the distance between the donor and acceptor.

And so, the application of FRET to the test kits and assay methods described herein is particularly appropriate because the presence of a ligand in the test sample will, through binding to its complimentary receptor, force a conformational change in the configuration of the reporter construct and therefore spatial relationship between donor and acceptor molecules or simply block energy transfer between the donor and acceptor molecules.

To validate their assay concept, Applicants used Cy3 and Cy5 labelled oligonucleotides. When annealed, the close proximity of Cy3 and Cy5 allows FRET to occur. In this particular configuration, light at 680nm is emitted when the annealed probe is excited at 540nm. The E_{FRET} is calculated from the donor (Cy3) and acceptor (Cy5) channels so 540nm for the donor (excitation) and 680nm (Cy5) for the acceptor (emission).

However, and importantly, there are numerous examples of other donor/acceptor molecules for application in FRET based sensors, such as those reviewed in Sapsford et al. (2006) Angew. Chem. Int. Ed. 45:4562-4588 and Zhong (2009) Anal. Bioanal. Chem. 394:47-59. While traditional FRET donor/acceptor molecules are organic dyes, modern nanotechnology produces materials like single metal nanoparticles and ionic nanocrystals that can be used in FRET application, offering significantly enhanced FRET effects and more flexible sensing platforms in bioanalysis.

By way of illustration, gold nanoparticles are excellent FRET-based quenchers because their plasmon resonance in the visible range makes them strong absorbers and scatterers, with large extinction coefficients of around 10⁵ cm⁻¹ M⁻¹. Indeed, gold nanoparticles have been used successfully in FRET applications with molecular beacons for the sensing of DNA (refer to Figure 16 of Sapsford *et al.* (2006) *ibid*)*.* The application of gold nanoparticles to these systems provided a 100-fold increase in sensitivity over previous dye combinations.

The importance of enhanced assay sensitivity is relevant to the Prototype Assays described in the Examples which follow, where the ΔE_{FRET} is in the order of <40%. In addition to the possibility of employing improved nanomaterials such as those reviewed in Sapsford et al. (2006) ibid, Applicants have identified that other techniques such as narrow scatter fluorescence filters may be used to compensate for lower ΔE_{FRET} than desired.

Quantum dots (QDs) have a number of unique optical properties that are advantageous in the development of bioanalyses based on fluorescence resonance energy transfer (FRET). Researchers have used QDs as energy donors in FRET schemes for the analysis of nucleic acids, proteins, proteases, haptens, and other small molecules. Existing FRET technologies can potentially be improved by using QDs as energy donors instead of conventional fluorophores. Superior brightness, resistance to photobleaching, greater optimization of FRET efficiency, and/or simplified multiplexing are possible with QD donors. The applicability of the Forster formalism to QDs and the feasibility of using QDs as energy acceptors are also reviewed in Algar & Krull (2008) Anal Bioanal Chem 391: 1609-1618.

Accordingly, the use of Quantum Dots (QD) in the test kits and assay methods is also envisaged. In direct comparison with organic dyes, several properties of QD stand out: size-tunable photoluminescent emission, broad absorption spectra and large Stokes shifts, which allows excitation of mixed QD populations at a wavelength far from its emission wavelength. For FRET applications in particular, this means that QDs could be size-tuned to give better spectral overlap with a particular acceptor dye. As the spectral overlap increases, there is a proportional increase in the value of R0, which togther with the high quantum yield of the QDs, permit FRET systems with longer separation distances. Accordingly, the reporter constructs described herein may be considerably lengthened. This is particularly important where the reporter constructs described herein are modified to include multiple copy number hormone response elements (e.g. 2xARE, 3xARE, 2xERE, 3xERE etc). Further, since QDs can be excited at almost any wavelength below their emission wavelength, an excitation wavelength can be chosen that corresponds to the absorption minimum of the acceptor so that direct excitation is minimized.

In other examples according to the test kits and assay methods described herein, the fluorescence generating and fluorescence quenching moieties include, but are not limited to: Pyrene; 7-Methoxycoumarin; Cascade Blue^{™}; Alexa Fluor^{®} 405; 7-Aminocoumarin-X; Alexa Fluor^{®} 350; Pacific Blue^{®}; Marina Blue^{®}; Dimethylaminocoumarin^{®}; BODIPY 493/503^{™}; BODIPY-FI-X^{™}; DTAF; 6-FAM (3') (Fluorescein); 6-FAM Amidite (Fluorescein); 6-FAM SE (Fluorescein); Dansyl-X; Oregon Green 500^{™}; Alexa Fluor^{®} 488; dT-FAM; dT-FAM (3'); Oregon Green 488^{™}; Rhodol Green^{™}; Oregon Green 514^{™}; Rhodamine Green-X^{™} (mixed isomer); NBD-X; TET Amidite; TET SE; CAL Fluor^{®} Gold 540; Alexa Fluor^{®} 430; Alexa Fluor^{®} 514; 2', 4', 5', 7'-Tetrabromosulfonefluorescein; BODIPY-FI Br2^{™}; 6-JOE; BODIPY-530/550^{™}; Alexa Fluor^{®} 532; HEX Amidite; HEX SE; Carboxyrhodamine 6G^{™}; CAL Fluor^{®} Orange 560; Cy3^{™}Amidite; Cy3^{™} SE; Alexa Fluor^{®} 555; BODIPY 558/568^{™}; BODIPY 564/570^{™}; BODIPY TMR-X^{™}; PyMPO; Quasar 570^{®}; Alexa Fluor^{®} 546; dT-TAMRA; TAMRA-X (Mixed Isomers); TAMRA-X (Single Isomer); Rhodamine Red-X^{™}; CAL Fluor^{®} Red 590; BODIPY 576/589^{™}; BODIPY 581/591^{™}; Alexa Fluor^{®} 568; Texas-Red-X^{™} (Mixed Isomers); Cy3.5^{™} Amidite; Cy3.5^{™} SE; Carboxy-X-Rhodamine^{™} (Mixed Isomers); Carboxy-X-Rhodamine^{™} (Single Isomer); CAL Fluor^{®} Red 610; BODIPY TR-XTM; Alexa Fluor^{®} 594; Alexa Fluor^{®} 610; CAL Fluor^{®} Red 635; Alexa Fluor^{®} 633; Alexa Fluor^{®} 647; Cy5^{™}Amidite; Cy5^{™} SE; Quasar 670^{®}; Carboxynaphthofluorescein (5 & 6 mixed esters); Alexa Fluor^{®} 660; Cy5.5^{™} Amidite; Cy5.5^{™}SE; Alexa Fluor^{®} 680; Alexa Fluor^{®} 700; Alexa Fluor^{®} 750; Black Hole Quenchers^{™} (BHQ); BHQ-0; BHQ-10; BHQ-1; BHQ-2; BHQ-3; Dabcyl; QSY-7; QSY 35; Eclipse; QSY 7; QSY 9; ElleQuencher; Iowa Black; QSY 21.

According to the test kits and assay methods described herein, when a receptor-ligand complex binds to the hormone response element, it blocks energy transfer between (e.g.) donor and acceptor molecules (e.g. fluorophores), thereby altering the level of static quenching or FRET quenching or a combination of both. The result is an increase or decrease in the amount of measurable fluorescence, reflecting the presence of a target ligand in a sample under investigation.

Accordingly, in another aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid reporter construct comprising:
   (a) a hormone response element that is capable of being bound by the receptor-ligand complex; and
   (b) a fluorescence generating moiety,
wherein, the presence of a ligand in the sample is detected by measuring a decrease in the fluorescence of the reporter construct caused by binding of the receptor-ligand complex to the hormone response element when the sample is combined with the test kit.

Accordingly, in another aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52; and
(iii) a nucleic acid reporter construct comprising:
   (a) a hormone response element that is capable of being bound by the receptor-ligand complex; and
   (b) a fluorescence generating moiety,
wherein, the presence of a ligand in the sample is detected by measuring a decrease in the fluorescence of the reporter construct caused by binding of the receptor-ligand complex to the hormone response element when the sample is combined with the test kit.

In another aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid reporter construct comprising:
   (a) a hormone response element that is capable of being bound by the receptor-ligand complex; and
   (b) a fluorescence generating moiety,
wherein, the presence of a ligand in the sample is detected by measuring an increase in the fluorescence of the reporter construct caused by binding of the receptor-ligand complex to the hormone response element when the sample is combined with the test kit.

In another aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52; and
(iii) a nucleic acid reporter construct comprising:
   (a) a hormone response element that is capable of being bound by the receptor-ligand complex; and
   (b) a fluorescence generating moiety,
wherein, the presence of a ligand in the sample is detected by measuring an increase in the fluorescence of the reporter construct caused by binding of the receptor-ligand complex to the hormone response element when the sample is combined with the test kit.

In other aspects according to the test kits and assay methods described herein, the reporter construct comprises discrete fluorescence generating and fluorescence quenching moieties, wherein the fluorescence generating moiety and the fluorescence quenching moiety are separated by a physical distance that permits either (i) Förster Resonance Energy Transfer (FRET) or (ii) static quenching between the fluorescence generating moiety and the fluorescence quenching moiety in the absence of a ligand.

In yet another aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid reporter construct comprising:
   (a) a hormone response element that is capable of being bound by the receptor-ligand complex;
   (b) a fluorescence generating moiety; and
   (c) a fluorescence quenching moiety,
wherein, the presence of a ligand in the sample is detected by measuring a change in fluorescence of the reporter construct caused by binding of the receptor-ligand complex to the hormone response element when the sample is combined with the test kit.

In yet another aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52; and
(iii) a nucleic acid reporter construct comprising:
   (a) a hormone response element that is capable of being bound by the receptor-ligand complex;
   (b) a fluorescence generating moiety; and
   (c) a fluorescence quenching moiety,
wherein, the presence of a ligand in the sample is detected by measuring a change in fluorescence of the reporter construct caused by binding of the receptor-ligand complex to the hormone response element when the sample is combined with the test kit.

In yet another aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid reporter construct comprising:
   (a) a hormone response element that is capable of being bound by the receptor-ligand complex;
   (b) a fluorescence generating moiety; and
   (c) a fluorescence quenching moiety,
   whereby (a) is positioned between (b) and (c)
wherein, the presence of a ligand in the sample is detected by measuring a change in fluorescence of the reporter construct caused by binding of the receptor-ligand complex to the hormone response element when the sample is combined with the test kit.

In yet another aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52; and
(iii) a nucleic acid reporter construct comprising:
   (a) a hormone response element that is capable of being bound by the receptor-ligand complex;
   (b) a fluorescence generating moiety; and
   (c) a fluorescence quenching moiety,
   whereby (a) is positioned between (b) and (c)
wherein, the presence of a ligand in the sample is detected by measuring a change in fluorescence of the reporter construct caused by binding of the receptor-ligand complex to the hormone response element when the sample is combined with the test kit.

In an example according to these and other aspects of the present disclosure, the presence of a ligand in the sample is detected by measuring a reduction or inhibition in either (i) Förster resonance energy transfer (FRET) and/or (ii) static quenching between the fluorescence generating moiety and the fluorescence quenching moiety.

In yet a further example according to this aspect of the present disclosure, the presence of a ligand in the sample is detected by measuring an increase in the amount of fluorescence generated by the reporter construct.

In yet another example according to this aspect of the present disclosure, the presence of a ligand in the sample is detected by measuring a decrease in the amount of fluorescence quenching by the reporter construct.

Accordingly, in yet another aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid reporter construct comprising:
   (a) a hormone response element that is capable of being bound by the receptor-ligand complex;
   (b) a fluorescence generating moiety; and
   (c) a fluorescence quenching moiety,
wherein, the presence of a ligand in the sample is detected by measuring an increase in fluorescence of the reporter construct caused by binding of the receptor-ligand complex to the hormone response element when the sample is combined with the test kit.

In yet another aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid reporter construct comprising:
   (a) a hormone response element that is capable of being bound by the receptor-ligand complex;
   (b) a fluorescence generating moiety comprising a quantum dot; and
   (c) a fluorescence quenching moiety comprising a gold nanoparticle,
wherein, the presence of a ligand in the sample is detected by measuring an increase in fluorescence of the reporter construct caused by binding of the receptor-ligand complex to the hormone response element when the sample is combined with the test kit.

In a further aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52; and
(iii) a nucleic acid reporter construct comprising:
   (a) a hormone response element that is capable of being bound by the receptor-ligand complex;
   (b) a fluorescence generating moiety; and
   (c) a fluorescence quenching moiety,
wherein, the presence of a ligand in the sample is detected by measuring an increase in fluorescence of the reporter construct caused by binding of the receptor-ligand complex to the hormone response element when the sample is combined with the test kit.

In yet a further aspect of the present disclosure there is described a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52; and
(iii) a nucleic acid reporter construct comprising:
   (a) a hormone response element that is capable of being bound by the receptor-ligand complex;
   (b) a fluorescence generating moiety comprising a quantum dot; and
   (c) a fluorescence quenching moiety comprising a gold nanoparticle,
wherein, the presence of a ligand in the sample is detected by measuring an increase in fluorescence of the reporter construct caused by binding of the receptor-ligand complex to the hormone response element when the sample is combined with the test kit.

In a further aspect of the present disclosure there is described a test kit for screening a sample for the presence of a ligand capable of eliciting a steroid hormone genomic response, the test kit comprising:
(i) a steroid hormone receptor that forms a receptor-ligand complex with a ligand from the sample; and
(ii) a nucleic acid reporter construct comprising:
   (a) a hormone response element that is capable of being bound by the receptor-ligand complex;
   (b) a fluorescence generating moiety; and
   (c) a fluorescence quenching moiety,
wherein, the presence of a ligand in the sample is detected by measuring a reduction or inhibition in fluorescence of the reporter construct caused by binding of the receptor-ligand complex to the nucleic acid response element when the sample is combined with the test kit.

The present disclosure further contemplates assay methods specifically involving fluorescence based reporter constructs.

Accordingly, in a further aspect of the present disclosure there is described an assay method for detecting a ligand in a sample, which ligand is capable of eliciting a steroid hormone genomic response, the method comprising the steps of:
(i) contacting a sample with:
   (a) a steroid hormone receptor that forms a receptor-ligand complex with a ligand from the test sample; and
   (b) a nucleic acid reporter construct comprising:
      i. a hormone response element that is bound by the receptor-ligand complex; and
      ii. a fluorescence generating moiety; and
(ii) measuring an increase in fluorescence of the reporter construct,
wherein, a measured increase in the fluorescence of the reporter construct reflects that a ligand has been detected in the sample.

In another aspect of the present disclosure there is described an assay method for detecting a ligand in a sample, which ligand is capable of eliciting a steroid hormone genomic response, the method comprising the steps of:
(i) contacting a sample with:
   (a) a steroid hormone receptor that forms a receptor-ligand complex with a ligand from the test sample; and
   (b) a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52; and
   (c) a nucleic acid reporter construct comprising:
      i. a hormone response element that is bound by the receptor-ligand complex; and
      ii. a fluorescence generating moiety; and
(ii) measuring an increase in fluorescence of the reporter construct,
wherein, a measured increase in the fluorescence of the reporter construct reflects that a ligand has been detected in the sample.

In a further aspect of the present disclosure there is described an assay method for detecting a ligand in a sample, which ligand is capable of eliciting a steroid hormone genomic response, the method comprising the steps of:
(i) contacting a sample with:
   (a) a steroid hormone receptor that forms a receptor-ligand complex with a ligand from the test sample; and
   (b) a nucleic acid reporter construct comprising:
      i. a hormone response element that is bound by the receptor-ligand complex; and
      ii. a fluorescence generating moiety comprising a quantum dot; and
      iii. a fluorescence quenching moeity comprising a gold nanoparticle; and
(ii) measuring an increase in fluorescence of the reporter construct,
wherein, a measured increase in the fluorescence of the reporter construct reflects that a ligand has been detected in the sample.

In a further aspect of the present disclosure there is described an assay method for detecting a ligand in a sample, which ligand is capable of eliciting a steroid hormone genomic response, the method comprising the steps of:
(i) contacting a sample with:
   (a) a steroid hormone receptor that forms a receptor-ligand complex with a ligand from the test sample; and
   (b) a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52; and
   (c) a nucleic acid reporter construct comprising:
      i. a hormone response element that is bound by the receptor-ligand complex; and
      ii. a fluorescence generating moiety comprising a quantum dot; and
      iii. a fluorescence quenching moeity comprising a gold nanoparticle; and
(ii) measuring an increase in fluorescence of the reporter construct,
wherein, a measured increase in the fluorescence of the reporter construct reflects that a ligand has been detected in the sample.

In a further aspect of the present disclosure there is described an assay method for detecting a ligand in a sample, which ligand is capable of eliciting a steroid hormone genomic response, the method comprising the steps of:
(i) contacting a sample with:
   (a) a steroid hormone receptor that forms a receptor-ligand complex with a ligand from the test sample; and
   (b) a nucleic acid reporter construct comprising:
      i. a hormone response element that is bound by the receptor-ligand complex; and
      ii. a fluorescence generating moiety; and
(ii) measuring a decrease in fluorescence of the reporter construct,
wherein, a measured decrease in the fluorescence of the reporter construct reflects that a ligand has been detected in the sample.

An important advantage conferred by the test kits and assay methods described herein is a significant reduction in the molecular complexity otherwise present in the cellular environment of yeast and mammalian cell reporter assays, or molecular complexity created through use of nuclear extracts for cell-free assays such as those described in WO2018/088852 which require RNA Polymerase II for performance. Reduced molecular complexity significantly enhances assay specificity by limiting non-specific activation of the hormone response element by steroid hormone receptor(s) in the absence of ligand. It is understood that hormone response elements (e.g. androgen response element) are not highly selective for their complimentary receptor (e.g. androgen receptor), and indeed may be activated by other steroid hormone receptors that may be present within the cell or nuclear extract. For example, in the case of the androgen response element, other non-androgen receptors in the Group II hormone receptor class such as progesterone receptor A, progesterone receptor B, glucocorticoid receptor and/or mineralocorticoid receptor. This in turn creates reduced assay specificity.

Indeed, the absence of molecular complexity created by a cellular environment or nuclear extract means that the test kits and assay methods described herein are highly selective for detection of their target ligands. Further, the ability to easily switch out (e.g.) a steroid hormone receptor and/or reporter construct comprising hormone response element creates versatility in the test kits and assay methods described herein for detection of other target ligands of interest.

Another important advantage conferred by the test kits and assay methods described herein is the unique ability to stoichiometrically define a biological reaction. For example, by precisely controlling the molecular relationship(s) between both essential and non-essential assay components, assay sensitivity may be significantly enhanced for the detection of a target ligand. In other words, the test kits and assay methods described herein may be configured to measure an *optimum* number of binding interactions between activated ligand-hormone receptor complexes and hormone response elements present within reporter constructs. In contrast, it is difficult, if not impossible, to replicate the same degree of control for cell-based reporter assays which have been configured to detect the presence of a ligand, since the total copy number of the gene or nucleic acid sequence encoding (e.g.) recombinant receptor and/or nucleic acid response element cloned into the cell cannot be predicted or controlled with any accuracy. It is also difficult to control for cell-free reporter assays based on nuclear extracts where it is difficult to determine exact amount of RNA polymerase II subunits present, cofactors, and other non-essential proteins.

These and other considerations are documented by the Examples which follow. By way of illustration, refer to Examples 1-2 when read in conjunction with Figures 1-3. Specifically, Applicants developed Prototype Assays involving detection of androgenic and estrogenic ligands as measured by a change in Förster Resonance Energy Transfer (i.e. ΔE_{FRET}) between Cy3 and Cy5 located on opposing termini of a nucleic acid reporter construct comprising a hormone response element (HRE). Applicants demonstrate that activated steroid hormone receptor (SHR) is most effective at binding to its complimentary HRE and inhibiting FRET between Cy3 and Cy5 when (i) the nucleic acid reporter construct is a minimum of 15 nucleotides in length (Fig. 1), (ii) the concentration of the nucleic acid reporter construct is 5-60 nM (Fig. 2) and (iii) the ratio of the steroid hormone receptor:nucleic acid reporter construct is between about 1:1 and 5:1 (Fig. 3). Indeed, Applicants observed a detrimental effect on ΔE_{FRET} where the number of nucleic acid reporter molecules exceeds the number of steroid hormone receptor molecules.

The results presented in Example 1, Table 1 directly compare the effect of manipulating the stoichiometry between the steroid hormone receptor and nucleic acid reporter construct, where for the same 15bp construct comprising the Cy3/Cy5 fluorophore combination at opposing termini, the ΔE_{FRET} increased from 15% to 34% in the presence of SHR ligand when the ratio of steroid hormone receptor to reporter construct/HRE was increased from 0.423 to 2.12. This represents a significant ΔE_{FRET} and therefore enhancement in assay sensitivity.

Accordingly, in a further example according to the test kits, assays and methods described herein, the ratio of steroid hormone receptor to nucleic acid reporter construct is between about 1:1 and about 5.0:1, and includes without limitation: 1.0:1, 1.1:1, 1.2:1, 1.3:1, 1.4:1, 1.5;1, 1.6:1, 1.7:1, 1.8:1, 1.9:1, 2.0:1, 2.1:1, 2.2:1, 2.3:1, 2.4:1, 2.5;1, 2.6:1, 2.7:1, 2.8:1, 2.9:1, 3.0:1, 3.1:1, 3.2:1, 3.3:1, 3.4:1, 3.5; 1, 3.6:1, 3.7:1, 3.8:1, 3.9:1, 4.0:1, 4.1:1, 4.2:1, 4.3:1, 4.4:1, 4.5;1, 4.6:1, 4.7:1, 4.8:1, 4.9:1 or 5.0:1. In a related example, the ratio of steroid hormone receptor to nucleic acid reporter construct is between about 2.0:1 and about 4.0:1, and includes without limitation 2.0:1, 2.1:1, 2.2:1, 2.3:1, 2.4:1, 2.5;1, 2.6:1, 2.7:1, 2.8:1, 2.9:1, 3.0:1, 3.1:1, 3.2:1, 3.3:1, 3.4:1, 3.5;1, 3.6:1, 3.7:1, 3.8:1, 3.9:1 or 4.0:1.

The skilled person would further appreciate that the molecular stiochiometry between the steriod hormone receptor and nucleic acid reporter construct comprising the hormone response element may vary depending on the component parts of the test kit/assay, as well as the nature of the sample to be tested. For example, the test kit/assay may by configured to detect ligands which bind to different steroid hormone receptors including, but not limited to, androgen receptor, estrogen receptor alpha, estrogen receptor beta, progesterone receptor A, progesterone receptor B, mineralocorticoid receptor and glucocorticoid receptor, where the ratio between the steroid hormone receptor and nuceic acid reporter construct may be (e.g.) between about 1:1 and about 20:1.

Accordingly, in yet another example according to the test kist and assay methods described herein:
(i) the test kit comprises an androgen receptor, and the ratio of androgen receptor to nucleic acid reporter construct comprising an androgen response element is between about 1:1 and about 20:1, including without limitation 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1 or 20:1;
(ii) the test kit comprises an estrogen receptor, and the ratio of estrogen receptor to nucleic acid comprising an estrogen response element is between about 1:1 and about 20:1, including without limitation 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1 or 20:1;
(iii) the test kit comprises a progesterone receptor, and the ratio of progsterone receptor to nucleic acid comprising a progesterone response element is between about 1:1 and about 20:1, including without limitation 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1 or 20:1;
(iv) the test kit comprises a mineralocorticoid receptor, and the ratio of mineralocorticoid receptor to nucleic acid comprising a mineralocorticoid response element is between about 1:1 and about 20:1, including without limitation 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1 or 20:1; and
(v) the test kit comprises a glucocorticoid receptor, and the ratio of glucocorticoid receptor to nucleic acid comprising a glucocorticoid response element is between about 1:1 and about 20:1, including without limitation 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1 or 20:1.

Notwithstanding the above considerations, care must be taken not to saturate the assay with too much receptor, since this in itself may create thermodynamic or kinetic barriers that prevent optimal binding of a receptor-ligand complex to its complimentary response element. According to the disclosure described herein, the skilled person could undertake routine experimentation to titrate an optimized conentration/range of steroid hormone receptor for performance of the test kits and assay methods described herein (e.g. refer to Example 1 which follows).

As previously mentioned, Applicants have further observed that the steroid hormone receptor retains *some* capacity to bind to and activate its corresponding nucleic acid response element in the absence of a ligand specific for its steroid hormone receptor. This phenomena is sometimes referred to as auto-activation of the hormone response element. While, by virtue of the reduced molecular complexity, the level of auto-activation is significantly diminished in the test kits and assays described herein, it may be desirable to determine a reference threshold (i.e. baseline signal as a result of auto-activation of the response element) in the absence of ligand to assist with a determination of absolute assay signal/readout in the presence of a sample containing a target ligand.

In a parallel approach to enhance assay specificity and performance, the test kit and assay methods described herein may be modified to include at least one steroid hormone receptor cofactor. The primary purpose of the cofactor is to hold the steroid hormone receptor in an inactive conformation, thereby preventing it from binding to and activating the hormone response element in the absence of a ligand.

When the test kit is contacted with a test sample, the presence of a ligand causes displacement of the cofactor and the ligand-bound receptor is then free to form a complex with a second ligand-bound receptor and the hormone response element.

Accordingly, in another example according to the test kits and assay methods described herein, the test kit or assay method further comprises at least one steroid hormone receptor cofactor.

In a related example, the steroid hormone receptor cofactor includes, without limitation, heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52.

In a further related example according to the test kits and assay methods described herein, the test kits or assay methods further comprise heat shock protein 90.

It will, however, be appreciated by a person skilled in the art that the presence of a steroid hormone receptor cofactor is not an essential feature of the test kits, assays and methods described herein. This is because an assay result may still be achieved in the absence of a cofactor. For example, the data presented in Example 2/Figure 4 illustrates there was *still* a measurable difference in signal between the ligand and non-ligand assay result (i.e. AR/T *versus* AR) in the absence of heat shock protein 90.

Indeed, there are further approaches in which to minimise the amount of signal generated by auto-activation of the hormone response element by non-liganded receptor, for example, by modifying the temperature at which an assay method is performed.

Applicants have observed a differential in the binding affinity/kinetics between ligand bound and non-ligand bound steroid hormone receptor for the nucleic acid response element. Accordingly, the test kits, assays and methods described herein may be performed at a temperature, or in a temperature range, that preferentially measures activation of a hormone response element by ligand-bound receptor over non-ligand bound receptor, thereby minimising any background signal generated by non-ligand bound receptor.

Accordingly, in yet another example according to this aspect of the present disclosure, performance of the test kit or assay method is carried out in a temperature range from about 25 °C to about 42 °C, and preferably from about 35 °C to about 37 °C.

The term "a temperature range from about 25 °C to about 42 °C" is intended to include any temperature from 25 °C to 42 °C and without limitation includes 25 °C, 26 °C, 27 °C, 28 °C, 29 °C, 30 °C, 31 °C, 32 °C, 33 °C, 34 °C, 35 °C, 36 °C, 37 °C, 38 °C, 39 °C, 40 °C, 41 °C and 42 °C. The skilled person would recognise that temeratures in the decimal point range may also be used. To further illustrate this point, "in a temperature range from about 35 °C to about 37 °C" includes, without limitation, 35.0 °C, 35.1 °C, 35.2 °C, 35.3 °C, 35.4 °C, 35.5 °C, 35.6 °C, 35.7 °C, 35.8 °C, 35.9 °C, 36.0 °C, 36.1 °C, 36.2 °C, 36.3 °C, 36.4 °C, 36.5 °C, 36.6 °C, 36.7 °C, 36.8 °C, 36.9 °C and 37.0 °C.

The Applicants further discovered that the stiochiometric relationship between the steroid hormone receptor cofactor and steriod hormone receptor may be manipulated to further enhance assay sensitivity. For example, according to the Androgen Assay Prototypes described in Examples 1 and 2, it was determined by the Applicants that AR is most effective at being activated by an AR-specific ligand and binding to ARE when the HSP90:AR ratio is about 1:1 to 5:1, and in particular about 2.5:1.

Accordingly, in a further example according to the test kits and assay methods described herein, the ratio of HSP90 to steroid hormone receptor is defined as between about 1:1 to about 5:1. This includes, without limitation, a ratio of HSP90 to steroid hormone receptor that is defined as 1.0:1, 1.1:1, 1.2:1, 1.3:1, 1.4:1, 1.5;1, 1.6:1, 1.7:1, 1.8:1, 1.9:1, 2.0:1, 2.1:1, 2.2:1, 2.3:1, 2.4:1, 2.5; 1, 2.6:1, 2.7:1, 2.8:1, 2.9:1, 3.0:1, 3.1:1, 3.2:1, 3.3:1, 3.4:1, 3.5; 1, 3.6:1, 3.7:1, 3.8:1, 3.9:1, 4.0:1, 4.1:1, 4.2:1, 4.3:1, 4.4:1, 4.5; 1, 4.6:1, 4.7:1, 4.8:1, 4.9:1 or 5.0:1.

The skilled person would, however, appreciate that the molecular stiochiometry between the steriod hormone receptor cofactor and steroid hormone receptor may vary depending on the composition of the test kit/assay. For example, the test kit/assay may by configured to detect ligands which bind to an estrogen receptor, including estrogen receptor alpha or estrogen receptor beta, and the ratio between the estrogen receptor cofactor and estrogen receptor may be (e.g.) between about 1:1 and about 10:1.

Accordingly, in yet another example according to the test kits and assay methods described herein:
(i) the test kit comprises an androgen receptor, and the ratio of androgen receptor cofactor to androgen receptor is between about 0.1:1 and about 10:1, including without limitation 1:1, 1.5:1, 2:1, 2.5:1, 3:1, 3.5:1, 4:1, 4.5:1, 5:1, 5.5:1, 6:1, 6.5:1, 7:1, 7.5:1, 8:1, 8.5:1, 9:1, 9.5:1 or 10:1;
(ii) the test kit comprises an estrogen receptor, and the ratio of estrogen receptor cofactor to estrogen receptor is between about 0.1:1 and about 10:1, including without limitation 1:1, 1.5:1, 2:1, 2.5:1, 3:1, 3.5:1, 4:1, 4.5:1, 5:1, 5.5:1, 6:1, 6.5:1, 7:1, 7.5:1, 8:1, 8.5:1, 9:1, 9.5:1 or 10:1;
(iii) the test kit comprises a progesterone receptor, and the ratio of progsterone receptor cofactor to progesterone receptor is between about 0.1:1 and about 10:1, including without limitation 1:1, 1.5:1, 2:1, 2.5:1, 3:1, 3.5:1, 4:1, 4.5:1, 5:1, 5.5:1, 6:1, 6.5:1, 7:1, 7.5:1, 8:1, 8.5:1, 9:1, 9.5:1 or 10:1;
(iv) the test kit comprises a mineralocorticoid receptor, and the ratio of mineralcorticoid receptor cofactor to mineralocorticoid receptor is between about 0.1:1 and about 10:1, including without limitation 1:1, 1.5:1, 2:1, 2.5:1, 3:1, 3.5:1, 4:1, 4.5:1, 5:1, 5.5:1, 6:1, 6.5:1, 7:1, 7.5:1, 8:1, 8.5:1, 9:1, 9.5:1 or 10:1; and
(v) the test kit comprises a glucocorticoid receptor, and the ratio of glucocorticoid receptor cofactor to glucocorticoid receptor is between about 0.1:1 and about 10:1, including without limitation 1:1, 1.5:1, 2:1, 2.5:1, 3:1, 3.5:1, 4:1, 4.5:1, 5:1, 5.5:1, 6:1, 6.5:1, 7:1, 7.5:1, 8:1, 8.5:1, 9:1, 9.5:1 or 10:1.

In a further example according to the test kits and assay methods described herein, the reporter construct comprises a single sequence copy of the hormone response element, or multiple sequence copies of the hormone response element. The term "multiple sequence copies" is intended to mean, without limitation, two, three, four, five, six, seven, eight, nine, 10, 11, 12, 13, 14, 15, 16, 17, 18, 19, 20, 21, 22, 23, 24, 25, 26, 27, 28, 29, 30, 31, 32 or more copies of the hormone response element. A person skilled in the art will recognize that the copy number of hormone response element sequences will be governed by the optimal signal to noise ratio, as determined by routine assay optimization.

The test kits and assay methods described herein are configured for detection of various ligands of both known and unknown structure which will bind to a steroid hormone receptor including androgen receptor (AR), estrogen receptor-a (ER-α), estrogen receptor-β (ER-β), progesterone receptor A (PRA), progesterone receptor B (PRB), mineralocorticoid receptor (MR) and glucocorticoid receptor (GR).

Examples of ligands known to bind androgen receptor include, without limitation, Testosterone, Dihydrotestosterone; anabolic androgenic steroids (AAS) including, but not limited to, TRENA, 17α-Trenbolone, 17β-Trenbolone, Trendione, Nandrolone, Boldenone, Altrenogest; selective androgen receptor modulators (SARMs) including, but not limited to, 93746, BMS-546929, LGD4033, ACP105, YK-11, Andarine, Ligandrol, Ostarine; Altrenogest.

Examples of ligands known to bind estrogen receptor alpha include, without limitation, Estradiol, Estrone, Estriol; selective estrogen receptor modulators including Raloxifene, Tamoxifen, Toremifene, Ospemifene, Lasofoxifene, Cyclofenil, Clomifene, Broparestrol, Basedoxifene, Anordrin; Phytoestrogens including but not limited to, dietary estrogens such as Polyphenols (Resveratrol), Flavanones (Eriodictyol, Hesperetin, Homoeriodictyol, Naringenin), Flavones (Apigenin, Luteolin, Tangeritin), Flavonols (Fisetin, Kaempferol, Myricetin, Pachypodol, Quercetin, Rhamnazin), Catechins (Proanthocyanides), Isoflavonoids (Isoflavones Biochanin A, Clycitein, Daidzein, Formononetin, Genistein), Isoflavans (Equol), Coumestans (Coumestrol); estrogen-like endocrine disruptive chemicals (EEDC) including, but not limited to, Dichlorodiphenyltrichloroethane (DDT), Dioxin, Polychlorinated Biphenyls (PCBs), Bisphenol A (BPA), Polybrominated Biphenyls (PBB), Phthalate Esters, Endosulfan, Atrazine, Zeranol; designer compounds such as Hydrazide Derivatives.

Examples of ligands known to bind estrogen receptor beta include, without limitation, all ligands which bind to estrogen receptor alpha, as well as, Diarylpropionitrile (DPN) and Wyeth-derived Benzoxazoles such as Way-659, Way-818 and Way-200070.

Examples of ligands known to bind progesterone receptor A and progesterone receptor B include, without limitation, Progesterone, Norethisterone, Levonorgestrel, Medroxyprogesterone Acetate, Megestrol Acetate, Dydrogesterone, Drospirenone; Selective Progesterone Receptor Modulators including Ulipristal Acetate, Telapristone Acetate, Vilaprisan, Asoprisnil, Asoprisnil Ecamate; Anti-Progestins including Mifepristone, Onapristone, Lilopritone and Gestrinone.

Examples of ligands known to bind mineralocorticoid receptor include, without limitation, Aldosterone; synthetic mineralocorticoids such as Fludrocortisone; antimineralocorticoids such as Spironolactone and Eplerenone; glucocorticoid receptor ligands such as those described below.

Examples of ligands known to bind glucocorticoid receptor include, without limitation dexamethasone, hydrocortisone, cortisone, prednisolone, methylprednisolone, prednisone, amcinonide, budesonide, desonide, fluocinonide, halcinonide, beclometasone, betamethasone, fluocortolone, halometasone, mometasone, or as antagonists mifepristone, and ketoconazole.

### Exemplary Assay Components & Constructs

In the Prototype Assays described in the Examples which follow, the nucleic acid reporter construct(s) comprising a hormone response element is assembled by annealing two single-stranded oligonucleotides, each labelled at the 5' end with a fluorophore. When the nucleic acid molecule is not bound by a SHR the two fluorophores can interact with each other and do so by passing electrons from the donor fluorophore to the acceptor fluorophore. This increases the energy state of the acceptor molecule which can be recorded by a fluorescence readout. Advantageously, the the acceptor fluorophore generates a fluorescence signal at a different wavelength to the donor molecule. When the nucleic acid molecule is bound by a steroid hormone receptor, electron transfer between the donor and the acceptor molecules is disrupted and the fluorescence readout is altered.

Steroid hormone receptors that are activated by a ligand present in a sample will dimerise and bind to its complimentary hormone response element. In the test kits and assays described herein, hormone response elements form part of the reporter construct, and a change in a physical property of the reporter construct may be used to reflect the presence of a ligand in a sample under investigation. Exemplary hormone response elements according to the present disclosure include: androgen response element (ARE), estrogen response element (ERE), progesterone response element (PRE), mineralcorticoid response element (MRE) and glucocorticoid response element (GRE).

As previously stated, the various hormone response elements incorporate binding motifs configured to selectively bind activated receptor-ligand complexes. For example, each of the androgen, estrogen, progesterone, mineralocorticoid and glucocorticoid response elements comprise imperfect dihexameric palindrome sequences which in their secondary structure orientations facilitate binding of dimerized ligand receptor complex (i.e. (HR-L)₂) via zinc finger binding motifs to its hormone response element.

In an example according to the test kits and assay methods described herein, the androgen response element comprises a DNA binding motif that selectively binds to an activated androgen receptor. In a related example, the DNA binding motif binds to a dimer of the ligand bound androgen receptor (i.e. (AR-L)₂; where "AR" is an androgen receptor and "L" is a ligand). In a related example, the DNA binding motif contains an imperfect dihexameric palindrome to create binding specificity between the activated androgen receptor and associated response element.

In an example according to the test kits and assay methods described herein, the estrogen response element comprises a DNA binding motif that selectively binds to an activated estrogen receptor. In a related example, the DNA binding motif binds to a dimer of the ligand bound estrogen receptor (i.e. (ER-L)₂; where "ER" is an estrogen receptor selected from ER-a or ER-β). In a further related example, the DNA binding motif contains an imperfect dihexameric palindrome to create binding specificity between the activated estrogen receptor and associated response element.

In an example according to the test kits and assay methods described herein, the progesterone response element comprises a DNA binding motif that selectively binds to an activated progesterone receptor. In a related example, the DNA binding motif binds to a dimer of the ligand bound progesterone receptor (i.e. (PR-L)₂; where "PR" is a progesterone receptor selected from PRA or PRB). In a further related example, the DNA binding motif contains an imperfect dihexameric palindrome to create binding specificity between the activated progesterone receptor and associated response element.

In an example according to the test kits and assay methods described herein, the mineralocorticoid response element comprises a DNA binding motif that selectively binds to an activated mineralocorticoid receptor. In a related example, the DNA binding motif binds to a dimer of the ligand bound mineralocorticoid receptor (i.e. (MR-L)₂; where "MR" is a mineralocorticoid receptor). In a further related example, the DNA binding motif contains an imperfect dihexameric palindrome to create binding specificity between the activated mineralocorticoid receptor and associated response element.

In an example according to the test kits and assay methods described herein, the glucocorticoid response element comprises a DNA binding motif that selectively binds to an activated glucocorticoid receptor. In a related example, the DNA binding motif binds to a dimer of the ligand bound glucocorticoid receptor (i.e. (GR-L)₂; where "GR" is a glucocorticoid receptor). In a further related example, the DNA binding motif contains an imperfect dihexameric palindrome to create binding specificity between the activated glucocorticoid receptor and associated response element.

Detection of a ligand that binds to and activates an androgen receptor, such as Testosterone, Dihydrotestosterone, synthetic steroid hormones (i.e. AAS) and selective androgen receptor modulators (i.e. SARMs) and phyto-or xenoandrogens, requires test kits/assays comprising an androgen receptor together with an androgen response element capable of binding to an activated androgen receptor-ligand complex.

Biochemical studies and the crystal structure of the dimerised AR DNA binding domain bound to the double-stranded ARE DNA show a consensus sequence of 15 nucleotides that includes three non-specified nucleotides in the centre of the binding site are not critical for molecular recognition of ARE by AR.

Accordingly, in an example according to the test kits and assay methods described herein, the androgen response element comprises or consist in the sequence 5'-AGAACAnnnTGTTCT-3' (SEQ ID NO: 1), where n is any nucleic acid base selected from G, C, T or A. Its complimentary antisense sequence is defined as 5'-AGAACAnnnTGTTCT-3' (SEQ ID NO: 2), where n represents the base that is complementary to SEQ ID NO: 1 based on a sequence alignment between SEQ ID Nos: 1 and 2 (i.e. A = T; T = A; G = C; C = G).

In another example according to the test kits and assay methods described herein, the androgen response element comprises or consist in the sequence 5'-GGTACAnnnTGTTCT-3' (SEQ ID NO: 3), where n is any nucleic acid base selected from G, C, T or A. Its complimentary antisense sequence is defined as 5'-AGAACAnnnTGTACC-3' (SEQ ID NO: 4), where n represents the base that is complementary to SEQ ID NO: 3 based on a sequence alignment between SEQ ID Nos: 3 and 4 (i.e. A = T; T = A; G = C; C = G).

Detection of a ligand that binds to and activates an estrogen receptor, such as Estradiol, Estrone, other estrogen-like steroid hormones including phyto- and xenoestrogens and selective estrogen receptor modulators, requires test kits/assays comprising either an estrogen receptor alpha (ER-α) or estrogen receptor beta (ER-β) together with an estrogen response element capable of binding to an activated estrogen receptor-ligand complex.

In an example according to the test kits and assay methods described herein, the estrogen response element comprises or consist in the sequence 5'-AGGTCAnnnTGACCT-3' (SEQ ID NO: 5), where n is any nucleic acid base selected from G, C, T or A. Its complimentary antisense sequence is defined as 5'-AGGTCAnnnTGACCT-3' (SEQ ID NO: 6), where n represents the base that is complementary to SEQ ID NO: 5 based on a sequence alignment between SEQ ID Nos: 5 and 6 (i.e. A = T; T = A; G = C; C = G).

Detection of a ligand that binds to and activates a progesterone receptor, such as Progesterone, Norethisterone, Levonorgestrel, other progesterone-like steroid hormones (i.e. PAS) and selective progesterone receptor modulators (i.e. SPRM), requires test kits/assays comprising either an progesterone receptor A (PRA) or progesterone receptor B (PRB) together with an progesterone response element capable of binding to an activated progesterone receptor-ligand complex.

In an example according to the test kits and assay methods described herein, the progesterone response element comprises or consist in the sequence 5'-GGTACAAACTGTTCT-3' (SEQ ID NO: 7). Its complimentary antisense sequence is defined as 5'-AGAACAGTTTGTACC-3' (SEQ ID NO: 8).

Detection of a ligand that binds to and activates a mineralocorticoid receptor, such as Aldosterone, synthetic mineralocorticoids such as Fludrocortisone and antimineralocorticoids such as Spironolactone and Eplerenone, requires test kits/assays comprising a mineralocorticoid receptor together with an mineralocorticoid response element capable of binding to an activated mineralocorticoid receptor complex.

In an example according to the test kits and assay methods described herein, the mineralocorticoid response element comprises or consist in the sequence 5'-AGAACAnAATGTTCT-3' (SEQ ID NO: 9), where n is any nucleic acid base selected from G, C, T or A. Its complimentary antisense sequence is defined as 5'-AGAACATTnTGTTCT-3' (SEQ ID NO: 10), where n represents the base that is complementary to SEQ ID NO: 9 based on a sequence alignment between SEQ ID NO: 9 and 10 (i.e. A = T; T = A; G = C; C = G).

Detection of a ligand that binds to and activates a glucocorticoid receptor, such as, Cortisol, Dexamethasone and 11-Dihydrocorticosterone requires test kits/assays comprising a glucocorticoid receptor together with an glucocorticoid response element capable of binding to an activated glucocorticoid receptor-ligand complex.

In an example according to the test kits and assay methods described herein, the glucocorticoid response element comprises or consist in the sequence 5'-AGAACAnAATGTTCT-3' (SEQ ID NO: 9), where n is any nucleic acid base selected from G, C, T or A. Its complimentary antisense sequence is defined as 5'-AGAACATTnTGTTCT-3' (SEQ ID NO: 10), where n represents the base that is complementary to SEQ ID NO: 9 based on a sequence alignment between SEQ ID NO: 9 and 10 (i.e. A = T; T = A; G = C; C = G).

In another example according to the present disclosure, the test kits and/or assay methods are configured to detect ligands that bind to an androgen receptor, and exemplary nucleic acid reporter constructs include, without limitation.

**Table 1: Fluorophore/Quencher reporter constructs comprising androgen response element(s)**

| **CONSTRUCT** | **SEQUENCE** | **SEQ IDENTIFIER** |
|---|---|---|
| 1 | **F**-5'-GGTACAnnnTGTTCT-3' | SEQ ID NO: 11 |
| | 3'-CCATGTnnnACAAGA-5'-**F** | SEQ ID NO: 12 |
| 2 | **F-**5'-GGTACGCATGTTCT-3' | SEQ ID NO: 13 |
| | 3'-CCATCTCGTACAAGA-5'-**F** | SEQ ID NO: 14 |
| 3 | **F**-5'-GGTACAnnnTGTTCT-3' | SEQ ID NO: 15 |
| | 3'-CCATGTnnnACAAGA-5'-**Q** | SEQ ID NO: 16 |
| 4 | **F-**5'-GGTACAGCATGTTCT-3' | SEQ ID NO: 17 |
| | 3'-CCATGTCGTACAAGA-5'**-Q** | SEQ ID NO: 18 |
| 5 | **F**-5'-ATTTTATTTAATATAATGGTACAnnnTGTTCTATTATATTAAATAAAAT-3' -**Q** | SEQ ID NO: 19 |
| | 3'-CCATGTnnnACAAGA-5' | SEQ ID NO: 20 |
| 6 | **F**-5'-TTTATTTAATATAATGGTACAnnnTGTTCTATTATATTAAATAAA-3' -**Q** | SEQ ID NO: 21 |
| | 3'-CCATGTnnnACAAGA-5' | SEQ ID NO: 22 |
| 7 | **F**-5' -ATTTAATATAATGGTACAnnnTGTTCTATTATATTAAAT-3' -**Q** | SEQ ID NO: 23 |
| | 3' -CCATGTnnnACAAGA-5' | SEQ ID NO: 24 |
| 8 | **F**-5' -TAATATAATGGTACAnnnTGTTCTATTATATTA-3' -**Q** | SEQ ID NO: 25 |
| | 3' -CCATGTnnnACAAGA-5' | SEQ ID NO: 26 |
| 9 | **F-**5'-TATAATGGTACAnnnTGTTCTATTATA-3'-**Q** | SEQ ID NO: 27 |
| | 3'-CCATGTnnnACAAGA-5' | SEQ ID NO: 28 |
| 10 | 5'-GGTACA**F**nnnTGTTCT-3' | SEQ ID NO: 29 |
| | 3'-CCATGT**Q**nnnACAAGA-5' | SEQ ID NO: 30 |
| 11 | 5'-GGTACAnnn**F**TGTTCT-3' | SEQ ID NO: 31 |
| | 3'-CCATGTnnn**Q**ACAAGA-5' | SEQ ID NO: 32 |
| 12 | 5'-GGTACAnnn**F**TGTTCT-3' | SEQ ID NO: 33 |
| | 3'-CCATGT**Q**nnnACAAGA-5' | SEQ ID NO: 34 |
| 13 | 5'-GGTACA**F**nnnTGTTCTGGTACAGCATGTTCT-3' | SEQ ID NO: 35 |
| | 3'-CCATGT**Q**nnnACAAGACCATGTCGTACAAGA-5' | SEQ ID NO: 36 |
| | | |
| 14 | 5'-GGTACAnnn**F**TGTTCTGGTACAGCATGTTCT-3' | SEQ ID NO: 37 |
| | 3'-CCATGT**Q**nnnACAAGACCATGTCGTACAAGA-5' | SEQ ID NO: 38 |
| 15 | 5'-GGTACAnnn**F**TGTTCTGGTACAAGCTGTTCT-3' | SEQ ID NO: 39 |
| | 3'-CCATGTnnn**Q**ACAAGACCATGTCGTACAAGA-5' | SEQ ID NO: 40 |
| 16 | **F**-5'-AGAACAnnnTGTTCT-3' | SEQ ID NO: 41 |
| | 3'-TCTTGTnnnACAAGA-5'-**F** | SEQ ID NO: 42 |
| 17 | **F-**5'-AGAACAGCATGTTCT-3' | SEQ ID NO: 43 |
| | 3'-TCTTGTCGTACAAGA-5'**-F** | SEQ ID NO: 44 |
| 18 | **F**-5'-AGAACAnnnTGTTCT-3' | SEQ ID NO: 45 |
| | 3'-TCTTGTnnnACAAGA-5'-**Q** | SEQ ID NO: 46 |
| 19 | **F-**5'-AGAACAGCATGTTCT-3' | SEQ ID NO: 47 |
| | 3'-TCTTGTCGTACAAGA-5'**-Q** | SEQ ID NO: 48 |
| 20 | **F**-5'-ATTTTATTTAATATAATAGAACAnnnTGTTCTATTATATTAAATAAAAT-3'-**Q** | SEQ ID NO: 49 |
| | 3'-TCTTGTnnnACAAGA-5' | SEQ ID NO: 50 |
| 21 | **F**-5'-TTTATTTAATATAATAGAACAnnnTGTTCTATTATATTAAATAAA-3'-**Q** | SEQ ID NO: 51 |
| | 3'-TCTTGTnnnACAAGA-5' | SEQ ID NO: 52 |
| 22 | **F**-5'-ATTTAATATAATAGAACAnnnTGTTCTATTATATTAAAT-3'-**Q** | SEQ ID NO: 53 |
| | 3'-TCTTGTnnnACAAGA-5' | SEQ ID NO: 54 |
| 23 | **F-**5'-TAATATAATAGAACAnnnTGTTCTATTATATTA-3'-**Q** | SEQ ID NO: 55 |
| | 3'-TCTTGTnnnACAAGA-5' | SEQ ID NO: 56 |
| 24 | **F-**5'-TATAATAGAACAnnnTGTTCTATTATA-3'**-Q** | SEQ ID NO: 57 |
| | 3'-TCTTGTnnnACAAGA-5' | SEQ ID NO: 58 |
| 25 | 5'-AGAACA**F**nnnTGTTCT-3' | SEQ ID NO: 59 |
| | 3'-TCTTGT**Q**nnnACAAGA-5' | SEQ ID NO: 60 |
| 26 | 5'-AGAACAnnn**F**TGTTCT-3' | SEQ ID NO: 61 |
| | 3'-TCTTGT**Q**nnnACAAGA-5' | SEQ ID NO: 62 |
| 27 | 5'-AGAACAnnn**F**TGTTCT-3' | SEQ ID NO: 63 |
| | 3'-TCTTGTnnn**Q**ACAAGA-5' | SEQ ID NO: 64 |
| 28 | 5'-AGAACA**F**nnnTGTTCTAGAACAGCATGTTCT -3' | SEQ ID NO: 65 |
| | 3'-TCTTGT**Q**nnnACAAGATCTTGTCGTACAAGA -5' | SEQ ID NO: 66 |
| 29 | 5'-AGAACAnnn**F**TGTTCTAGAACAGCATGTTCT -3' | SEQ ID NO: 67 |
| | 3'-TCTTGT**Q**nnnACAAGATCTTGTCGTACAAGA -5' | SEQ ID NO: 68 |
| 30 | 5'-AGAACAnnn**F**TGTTCTAGAACAGCATGTTCT -3' | SEQ ID NO: 69 |
| | 3'-TCTTGTnnn**Q**ACAAGATCTTGTCGTACAAGA -5' | SEQ ID NO: 70 |

**Table 2: Fluorophore/Quencher reporter constructs comprising estrogen response element(s)**

| **CONSTRUCT** | **SEQUENCE** | **SEQ IDENTIFIER** |
|---|---|---|
| 31 | **F**-5'-AGGTCAnnnTGACCT-3' | SEQ ID NO: 71 |
| | 3'-TCCAGTnnnACTGGA-5'-**F** | SEQ ID NO: 72 |
| 32 | **F**-5'-AGGTCAGCATGACCT-3' | SEQ ID NO: 73 |
| | 3'-TCCAGTCGTACTGGA-5'-**F** | SEQ ID NO: 74 |
| 33 | **F**-5'-AGGTCAnnnTGACCT-3' | SEQ ID NO: 75 |
| | 3'-TCCAGTnnnACTGGA-5'-**Q** | SEQ ID NO: 76 |
| 34 | **F**-5'-AGGTCAGCATGACCT-3' | SEQ ID NO: 77 |
| | 3'-TCCAGTCGTACTGGA-5'-**Q** | SEQ ID NO: 78 |
| 35 | **F**-5'-ATTTTATTTAATATAATAGGTCAnnnTGACCTATTATATTAAATAAAAT-3'-**Q** | SEQ ID NO: 79 |
| | 3'- TCCAGTnnnACTGGA -5' | SEQ ID NO: 80 |
| 36 | **F**-5'-TTTATTTAATATAATAGGTCAnnnTGACCTATTATATTAAATAAA-3'-**Q** | SEQ ID NO: 81 |
| | 3'- TCCAGTnnnACTGGA -5' | SEQ ID NO: 82 |
| 37 | **F**-5'-ATTTAATATAATAGGTCAnnnTGACCTATTATATTAAAT-3'-**Q** | SEQ ID NO: 83 |
| | 3'- TCCAGTnnnACTGGA -5' | SEQ ID NO: 84 |
| 38 | **F**-5'-TAATATAATAGGTCAnnnTGACCTATTATATTA-3'**-Q** | SEQ ID NO: 85 |
| | 3'- TCCAGTnnnACTGGA -5' | SEQ ID NO: 86 |
| 39 | **F**-5'-TATAATAGGTCAnnnTGACCTATTATA-3'-**Q** | SEQ ID NO: 87 |
| | 3'- TCCAGTnnnACTGGA -5' | SEQ ID NO: 88 |
| 40 | 5'-AGGTCA**F**nnnTGACCT-3' | SEQ ID NO: 89 |
| | 3'-TCCAGT**Q**nnnACTGGA-5' | SEQ ID NO: 90 |
| 41 | 5'-AGGTCAnnn**F**TGACCT-3' | SEQ ID NO: 91 |
| | 3'-TCCAGT**Q**nnnACTGGA-5' | SEQ ID NO: 92 |
| 42 | 5'-AGGTCAnnn**F**TGACCT-3' | SEQ ID NO: 93 |
| | 3'-TCCAGTnnn**Q**ACTGGA-5' | SEQ ID NO: 94 |
| 43 | 5'-AGGTCA**F**nnnTGACCTAGGTCAGCATGACCT-3' | SEQ ID NO: 95 |
| | 3'-TCCAGT**Q**nnnACTGGATCCAGTCGTACTGGA-5' | SEQ ID NO: 96 |
| 44 | 5'-AGGTCAnnn**F**TGACCTAGGTCAGCATGACCT-3' | SEQ ID NO: 97 |
| | 3'-TCCAGT**Q**nnnACTGGATCCAGTCGTACTGGA-5' | SEQ ID NO: 98 |
| 45 | 5'-AGGTCAnnn**F**TGACCTAGGTCAGCATGACCT-3' | SEQ ID NO: 99 |
| | 3'-TCCAGTnnn**Q**ACTGGATCCAGTCGTACTGGA-5' | SEQ ID NO: 100 |

**Table 3: Fluorophore/Quencher reporter constructs comprising progesterone response element(s)**

| **CONSTRUCT** | **SEQUENCE** | **SEQ IDENTIFIER** |
|---|---|---|
| 46 | **F**-5'-GGTACAnnnTGTTCT-3' | SEQ ID NO: 101 |
| | 47 3'-CCATGTnnnACAAGA-5'-**F** | SEQ ID NO: 102 |
| 47 | **F**-5'-GGTACACATGTTCT-3' | SEQ ID NO: 103 |
| | 3'-CCATGTGTACAAGA-5'-**F** | SEQ ID NO: 104 |
| 48 | **F**-5'-GGTACAnnnTGTTCT-3' | SEQ ID NO: 105 |
| | 3'-CCATGTnnnACAAGA-5'-**Q** | SEQ ID NO: 106 |
| 49 | **F**-5'-GGTACAGCATGTTCT-3' | SEQ ID NO: 107 |
| | 3'-CCATGTCGTACAAGA-5'-**Q** | SEQ ID NO: 108 |
| 50 | **F**-5'-ATTTTATTTAATATAATGGTACAnnnTGTTCTATTATATTAAATAAAAT-3'-**Q** | SEQ ID NO: 109 |
| | 3'-CCATGTnnnACAAGA-5' | SEQ ID NO: 110 |
| 51 | **F**-5'-TTTATTTAATATAATGGTACAnnnTGTTCTATTATATTAAATAAA-3'-**Q** | SEQ ID NO: 111 |
| | 3'-CCATGTnnnACAAGA-5' | SEQ ID NO: 112 |
| 52 | **F**-5'-ATTTAATATAATGGTACAnnnTGTTCTATTATATTAAAT-3'-**Q** | SEQ ID NO: 113 |
| | 3'-CCATGTnnnACAAGA-5' | SEQ ID NO: 114 |
| 53 | **F**-5'-TAATATAATGGTACAnnnTGTTCTATTATATTA-3'-**Q** | SEQ ID NO: 115 |
| | 3'-CCATGTnnnACAAGA-5' | SEQ ID NO: 116 |
| 54 | **F**-5'-TATAATGGTACAnnnTGTTCTATTATA-3'-**Q** | SEQ ID NO: 117 |
| | 3'-CCATGTnnnACAAGA-5' | SEQ ID NO: 118 |
| 55 | 5'-GGTACA**F**nnnTGTTCT-3' | SEQ ID NO: 119 |
| | 3'-CCATGT**Q**nnnACAAGA-5' | SEQ ID NO: 120 |
| 56 | 5'-GGTACAnnn**F**TGTTCT-3' | SEQ ID NO: 121 |
| | 3'-CCATGT**Q**nnnACAAGA-5' | SEQ ID NO: 122 |
| 57 | 5'-GGTACAnnn**F**TGTTCT-3' | SEQ ID NO: 123 |
| | 3'-CCATGTnnn**Q**ACAAGA-5' | SEQ ID NO: 124 |
| 58 | 5'-GGTACA**F**nnnTGTTCTGGTACAGCATGTTCT-3' | SEQ ID NO: 125 |
| | 3'-CCATGT**Q**nnnACAAGACCATGTCGTACAAGA-5' | SEQ ID NO: 126 |
| 59 | 5'-GGTACAnnn**F**TGTTCTGGTACAGCATGTTCT-3' | SEQ ID NO: 127 |
| | 3'-CCATGT**Q**nnnACAAGACCATGTCGTACAAGA-5' | SEQ ID NO: 128 |
| 60 | 5'-GGTACAnnn**F**TGTTCTGGTACATGCATGTTCT-3' | SEQ ID NO: 129 |
| | 3'-CCATGTnnn**Q**ACAAGACCATGTCGTACAAGA-5' | SEQ ID NO: 130 |

**Table 4: Fluorophore/Quencher reporter constructs comprising mineralocorticoid/glucocorticoid response element(s)**

| **CONSTRUCT** | **SEQUENCE** | **SEQ IDENTIFIER** |
|---|---|---|
| 61 | F-5'-AGAACAnnnTGTTCT-3' | SEQ ID NO: 131 |
| | 3'-TCTTGTnnnACAAGA-5' -F | SEQ ID NO: 132 |
| 62 | F-5'-AGAACAGCATGTTCT-3' | SEQ ID NO: 133 |
| | 3'-TCTTGTCGTACAAGA-5'-F | SEQ ID NO: 134 |
| 63 | F-5'-AGAACAnnnTGTTCT-3' | SEQ ID NO: 135 |
| | 3'-TCTTGTnnnACAAGA-5'-Q | SEQ ID NO: 136 |
| 64 | F-5'-AGAACAGCATGTTCT-3' | SEQ ID NO: 137 |
| | 3'-TCTTGTCGTACAAGA-5'-Q | SEQ ID NO: 138 |
| 65 | F-5'-ATTTTATTTAATATAATAGAACAnnnTGTTCTATTATATTAAATAAAAT-3'-Q | SEQ ID NO: 139 |
| | 3'-TCTTGTnnnACAAGA-5' | SEQ ID NO: 140 |
| 66 | F-5'-TTTATTTAATATAATAGAACAnnnTGTTCTATTATATTAAATAAA-3'-Q | SEQ ID NO: 141 |
| | 3'-TCTTGTnnnACAAGA-5' | SEQ ID NO: 142 |
| 67 | F-5'-ATTTAATATAATAGAACAnnnTGTTCTATTATATTAAAT-3'-Q | SEQ ID NO: 143 |
| | 3'-TCTTGTnnnACAAGA-5' | SEQ ID NO: 144 |
| 68 | F-5'-TAATATAATAGAACAnnnTGTTCTATTATATTA-3'-Q | SEQ ID NO: 145 |
| | 3'-TCTTGTnnnACAAGA-5' | SEQ ID NO: 146 |
| 69 | F-5'-TATAATAGAACAnnnTGTTCTATTATA-3'-Q | SEQ ID NO: 147 |
| | 3'-TCTTGTnnnACAAGA-5' | SEQ ID NO: 148 |
| 70 | 5'-AGAACAFnnnTGTTCT-3' | SEQ ID NO: 149 |
| | 3'-TCTTGTQnnnACAAGA-5' | SEQ ID NO: 150 |
| 71 | 5'-AGAACAnnnFTGTTCT-3' | SEQ ID NO: 151 |
| | 3'-TCTTGTQnnnACAAGA-5' | SEQ ID NO: 152 |
| 72 | 5'-AGAACAnnnFTGTTCT-3' | SEQ ID NO: 153 |
| | 3'-TCTTGTnnnQACAAGA-5' | SEQ ID NO: 154 |
| 73 | 5'-AGAACAFnnnTGTTCTAGAACAGCATGTTCT-3' | SEQ ID NO: 155 |
| | 3'-TCTTGTQnnnACAAGATCTTGTCGTACAAGA-5' | SEQ ID NO: 156 |
| 74 | 5'-AGAACAnnnFTGTTCTAGAACAGCATGTTCT-3' | SEQ ID NO: 157 |
| | 3'-TCTTGTQnnnACAAGATCTTGTCGTACAAGA-5' | SEQ ID NO: 158 |
| 75 | 5'-AGAACAnnnFTGTTCTAGAACAGCATGTTCT-3' | SEQ ID NO: 159 |
| | 3'-TCTTGTnnnQACAAGATCTTGTCGTACAAGA-5' | SEQ ID NO: 160 |

### Multiplexed Assay Systems

The present disclosure further contemplates multiplexed assays configured to detect two or more steroid hormone genomic responses from the *same* test sample.

To further illustrate the relevance of multiplexed systems, in certain circumstances it would be useful for a clinician investigating, for example, the hormonal status of a subject to know both the androgenic and estrogenic levels/activity in the subject.

The side-by-side detection of androgenic and estrogenic ligands from the same test sample is possible because a ligand which binds to an androgen receptor will not bind to and activate an estrogen receptor present in the same assay; conversely a ligand which binds to an estrogen receptor which not bind to and activate an androgen receptor also present in the same assay. This is because androgen and estrogen receptors belong to different steroid hormone receptor classes, and so there is no 'cross-talk' in terms of receptor activation. And so, multiplexed assays have been developed to detect both androgenic and estrogenic ligands from the same sample.

Accordingly, in another aspect of the present disclosure there is described a test kit for screening a sample for the side-by-side detection of an androgenic ligand and/or an estrogenic ligand, the test kit comprising:
(i) an androgen receptor, wherein the androgen receptor is capable of forming an androgen receptor-ligand complex with a complimentary ligand from the sample; and
(ii) a first nucleic acid reporter construct comprising:
   (a) an androgen response element that is capable of being bound by the androgen receptor-ligand complex; and
   (b) a fluorescence generating moiety; and
(iii) an estrogen receptor, wherein the estrogen receptor is capable of forming an estrogen receptor-ligand complex with a complimentary ligand from the sample; and
(iv) a second nucleic acid reporter construct comprising:
   (a) an estrogen response element that is capable of being bound by the estrogen receptor-ligand complex; and
   (b) a fluorescence generating moiety;

wherein, the first and second reporter constructs are different,
and wherein, the presence of an androgenic ligand in the sample is detected by measuring a change in fluorescence of the first reporter construct caused by binding of the androgen receptor-ligand complex to the response element when the sample is combined with the test kit,
and wherein the presence of an estrogenic ligand in the sample is detected by measuring a change in fluorescence of the second reporter construct caused by binding of the estrogen receptor-ligand complex to the response element when the sample is combined with the test kit.

In an example according to this aspect of the present disclosure, the test kit further comprises heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52.

In another example according to this aspect of the present disclosure, the fluorescence generating moiety comprises a fluorescence generating molecule and a fluorescence quenching molecule.

In a related example according to this aspect of the present disclosure, the fluorescence generating moiety comprises a fluorescence generating molecule comprising a quantum dot and a fluorescence quenching molecule comprising a gold nanoparticle.

In an example according to this aspect of the present disclosure, the first and second nucleic acid molecules are discrete molecules.

In yet another example according to this aspect of the present disclosure, the first nucleic acid molecule comprises a sequence defined by 5'-AGAACAnnnTGTTCT-3', where n is any nucleic acid base selected from G, C, T or A (SEQ ID NO: 1). In a further example the first nucleic acid molecule comprises any one of SEQ ID NOs: 11-70.

In a further example according to this aspect of the present disclosure, the second nucleic acid molecule comprises a sequence defined by 5'-AGGTCAnnnTGACCT-3', where n is any nucleic acid base selected from G, C, T or A (SEQ ID NO: 6). In a further example the first nucleic acid molecule comprises any one of SEQ ID NOs: 71-100.

In yet a further example according to this aspect of the present disclosure the first and second nucleic acid molecules are operably linked and comprise a sequence defined by AGAACAnnnTGTTCTnnnAGGTCAnnnTGACCT, where n is any nucleic acid base selected from G, C, T or A (SEQ ID NO: 160).

In yet a further example according to this aspect of the present disclosure the first and second nucleic acid molecules are operably linked and comprise a sequence defined by AGGTCAnnnTGACCTnnnAGAACAnnnTGTTCT, where n is any nucleic acid base selected from G, C, T or A (SEQ ID NO: 161).

Advantageously, the assays and test kits described herein are particularly suited for configuration in multiplexed systems because (i) their performance is possible in the absence of a cell-extract which otherwise contains naturally occuring ligands and/or steriod hormone receptors that interfere with the assay signal (e.g. ligand and receptor 'cross-talk' leads to autoactivation of the response element) and (ii) the simplicity of the assay systems described herein means that an existing assay or test kit may be routinely modified to include a second or subsequent receptor/report construct combination specific to detection of a second ligand with discrete signals generated by each reporter conveniently detected. To further illustrate this point, a multiplexed assay system according to the present disclosure may comprise, for example, an andogen specific reporter construct which, in the presence of androgen or an androgen-like ligand, would generate a reporter read-out that may be measured independently of the read-out generated by a reporter construct that is specific for the detection of estradiol in the same sample.

The skilled person would appreciate the advantages conferred by a lack of molecular complexity associated with the multiplexed systems of the present disclosure, and would recognise that detection of multiple *discrete* steroid hormone genomic responses (e.g. two, three, four, or more) from the same test sample is possible.

Accordingly, the test kits according to the present disclosure comprise at least one steriod hormone receptor and at least one nucleic acid molecule comprising at least one reporter construct.

Accordingly, the term "a steroid hormone receptor" according to the test kits and methods described herein is intended to mean "at least one steriod hormone receptor" and would include "two steroid hormone receptors" in the sense that two or more *different types* of sterioid hormone receptors may be present (e.g. and by way of illustration only, a steroid hormone receptor that binds testosterone and a steroid hormone receptor that binds estradiol).

Similarly, the term "a nucleic acid molecule [comprising a response element]" is intended to mean "at least one nucleic acid" in the sense that two or more discrete nucleic acid molecules may be present, each comprising a different response element and optionally a different reporter molecule. Alternatively, one nucleic acid molecule could be present that encodes for two or more *different types* of hormone response elements.

In an example according to this aspect of the present disclosure, the test kit comprises (i) an estrogen receptor and nucleic acid molecule comprising an estrogen response element, and (ii) an androgen receptor and nucleic acid molecule comprising an androgen response element.

In a related example, the nucleic acid molecule comprising an estrogen response element further comprises a first fluorescence generating moiety, and the nucleic acid molecule comprising the androgen response element further comprises a second fluorescence generating moiety, wherein the fluorescence signal generated by the first fluorescence generating moiety is discrete from the fluorescence signal generated by the second fluorescence generating moiety.

According to the multiplexed assays described, the first and second nucleic acid molecules are provided separately. Exemplary constructs for use in the multiplexed assays described herein include, but are not limited to, a reporter construct comprising an androgen response element selected from Table 5 and, separately, a reporter construct comprising an estrogen response element selected from Table 6. That is the multiplexed assay reaction mix comprises two discrete double stranded DNA reporter constructs.

**Table 5: Exemplary reporter constructs comprising ARE for use in a multiplexed assay**

| **Construct** | **Sequence** | **SEQ ID NO:** |
|---|---|---|
| 16 | **F**-5'-AGAACAnnnTGTTCT-3' | SEQ ID NO: 41 |
| | 3'-TCTTGTnnnACAAGA-5'-**F** | SEQ ID NO: 42 |
| 17 | **F**-5'-AGAACAGCATGTTCT-3' | SEQ ID NO: 43 |
| | 3'-TCTTGTCGTACAAGA-5'-**F** | SEQ ID NO: 44 |
| 18 | **F**-5'-AGAACAnnnTGTTCT-3' | SEQ ID NO: 45 |
| | 3'-TCTTGTnnnACAAGA-5'-**Q** | SEQ ID NO: 46 |
| 19 | **F**-5'-AGAACAGCATGTTCT-3' | SEQ ID NO: 47 |
| | 3'-TCTTGTCGTACAAGA-5'-**Q** | SEQ ID NO: 48 |

**Table 6: Exemplary reporter constructs comprising ERE for use in a multiplexed assay**

| **Construct** | **Sequence** | **SEQ ID NO:** |
|---|---|---|
| 31 | **F**-5'-AGGTCAnnnTGACCT-3' | SEQ ID NO: 71 |
| | 3'-TCCAGTnnnACTGGA-5'-**F** | SEQ ID NO: 72 |
| 32 | **F**-5'-AGGTCAGCATGACCT-3' | SEQ ID NO: 73 |
| | 3'-TCCAGTCGTACTGGA-5'-**F** | SEQ ID NO: 74 |
| 33 | **F**-5'-AGGTCAnnnTGACCT-3' | SEQ ID NO: 75 |
| | 3'-TCCAGTnnnACTGGA-5'-**Q** | SEQ ID NO: 76 |
| 34 | **F**-5'-AGGTCAGCATGACCT-3' | SEQ ID NO: 77 |
| | 3'-TCCAGTCGTACTGGA-5'-**Q** | SEQ ID NO: 78 |

Alternatively, according to the multiplexed assays described, the first and second nucleic acid molecules are operably linked. As such, exemplary constructs for use in the multiplexed assays described herein include, but are not limited to, a reporter construct comprising both an androgen response element and an estrogen response element selected from Table 7, below.

**Table 7: Exemplary reporter constructs comprising ARE+ERE for use in a multiplexed assay**

| **Construct** | **Sequence** | **SEQ ID NO:** |
|---|---|---|
| 76 | **F**-5'-AGAACAnnnTGTTCTnnnAGGTCAnnnTGACCT-3' | SEQ ID NO: 163 |
| | 3'-TCTTGTnnnACAAGAnnnTCCAGTnnnACTGGA-5'-**F** | SEQ ID NO: 164 |
| 77 | **F**-5'-AGAACAGCATGTTCTnnnAGGTCAGCATGACCT-3' | SEQ ID NO: 165 |
| | 3'-TCTTGTCGTACAAGAnnnTCCAGTCGTACTGGA-5'-**F** | SEQ ID NO: 166 |
| 78 | **F**-5'-AGAACAnnnTGTTCTnnnTCCAGTnnnACTGGA-5' | SEQ ID NO: 167 |
| | 3'-TCTTGTnnnACAAGATCCAGTnnnACTGGA-5'-**Q** | SEQ ID NO: 168 |
| 79 | **F**-5'-AGAACAGCATGTTCTnnnAGGTCAGCATGACCT-3' | SEQ ID NO: 169 |
| | 3'-TCTTGTCGTACAAGAnnnTCCAGTCGTACTGGA-5'-**Q** | SEQ ID NO: 170 |

### Utility of the Test Kits & Assays

Advantageously, the present disclosure describes activity based test kits, assays and methods that work fundamentally on the principle of steroid hormone receptor activation. By detecting steroid hormone receptor activation (with consequent binding to its hormone response element) by a target ligand present within a sample to be tested, the present disclosure conveniently describes cell-free and enzyme-free test kits, assays and methods that do not rely on structural knowledge of the ligand(s) being interrogated, can readily distinguish between the presence of biologically active and inactive ligands, and provide cost-effective, reliable and reproducible systems that do not require complex laboratory equipment or particular expertise to perform.

Accordingly, in another aspect of the present disclosure there is described a method for determining the doping status of an athlete, the method comprising combining a sample obtained from the athlete with a test kit as described herein and determining the doping status of an athlete.

In an example according to this aspect of the present disclosure, the sample obtained obtained from the athlete is a serum sample, a plasma sample or a urine sample.

In another example, the athlete is a human athlete or a non-human athlete selected from a horse, a camel or a dog.

In a further aspect of the present disclosure there is described an article of manufacture for screening a test sample for the presence of a ligand, which ligand is capable of activating a steroid hormone receptor and eliciting a genomic response in a cell, the article of manufacture comprising a test kit as described herein together with instructions for how to detect the presence of a ligand in the sample.

In yet a further aspect of the present disclosure there is described an article of manufacture for determining doping in an athlete, the article of manufacture comprising a test kit as described herein together with instructions for detecting the presence of a ligand in a sample derived from the athlete, wherein the presence of the ligand in the sample is indicative of doping in the athlete.

The various test kits and assays described herein each provide (i) a steroid hormone receptor inclusive of a ligand binding domain for binding a ligand that *may* be present in a sample to be tested and (ii) a nucleic acid response element comprising a protein binding domain which is bound by an activated steroid hormone receptor (or receptor-ligand complex; HR-L). The term "activated steroid hormone receptor" refers to a receptor-ligand complex, and may include various permutations of the HR-L structure (e.g. monomer, dimer, trimer *etc*). Importantly, the hormone response element contains binding motifs specific for the receptor-ligand complex. Accordingly, by combining the test kits and assays of the present disclosure with a sample of interest, detection of a ligand, which possesses the potential to bind to a steroid hormone receptor and elicit a steroid hormone genomic response, is possible.

The terms "receptor binding domain", "activated receptor binding domain", "hormone receptor binding domain", "activated hormone receptor binding domain", "receptor-ligand binding domain" and "hormone receptor-ligand binding domain" are used interchangeably to refer to the protein binding domain of the hormone response element that is bound by an activated hormone receptor or receptor-ligand complex, as defined herein.

In other examples, the disclosures described herein find utility in the detection of performance enhancing pro/drugs (e.g. anabolic steroids) used in human as well as non-human athletes including race horses and dogs. In other examples, the disclosures described herein have utility in screening foods and health food supplements for additives that may bind to a steroid hormone receptor and elicit a genomic response in a cell or do so following metabolic processing (i.e. in the case of so-called 'prodrugs').

The present disclosure further contemplates detection of one or more physiologically inactivate ligands from a test sample, which ligands are ultimately capable of activating steroid hormone receptors when converted to a physiologically active form. As such, the test kits, assays and methods as described herein further comprise steroid metabolism machinery that is capable of processing the ligand in such a way that it will activate its corresponding steroid hormone receptor. In this way, detection of physiologically inactive ligands (e.g. prohormones) from samples such as nutritional supplements is possible.

As such, the test kits, assays and methods described herein may further comprise steroid metabolism machinery sufficient to convert a ligand from a physiologically inactive form to a physiologically active form, or from a physiologically active form to a *more* physiologically active form or from a physiologically active form to a less physiologically active form, or from a physiologically active form to a physiologically inactive form. Only when the ligand is in a physiologically active form does it possess the ability to activate a steroid hormone receptor and elicit a genomic response. Accordingly, inclusion of steroid metabolism machinery in the test kits, assays and methods according to the present disclosure helps facilitate detection of physiologically inactive ligands from a test sample of interest, (e.g.) which ligands exist as pro-drugs (e.g. pro-hormones) and might otherwise evade detection using established methodologies. Furthermore, inclusion of steroid metabolism machinery in the test kits, assays and methods according to the present disclosure helps determine biological activity/potency of ligands necessary to show effect.

The test kits, assays and methods described herein may further comprise a detection means for detecting binding between the receptor-ligand complex and the response element contained within the nucleic acid, as defined.

The test kits and assays according to the present disclosure are cell-free. This is particularly important since the molecular complexity of the assay systems are significantly reduced. For example, the absence of (i) a cell membrane structure which has the potential to create a thermodynamic sink for steroid hormone molecules and (ii) endogenous steroid hormone metabolism observed with cell based systems, provides for an assay system with enhanced sensitivity. Further, and advantageously, according to the test kits, assays and methods described herein, the relative amounts of essential structural elements (e.g. steroid hormone receptor and nucleic acid response element inclusive of one or more activated receptor binding domains) may be precisely controlled to provide enhanced assay functionality and increased sensitivity.

According to the methods described herein, the test result may be compared to a reference threshold in order to determine the absolute level of signal generated by a ligand present in a test sample. Indeed, Applicants observed non-specific binding and/or activation of the response element by non-ligand bound receptor. Accordingly, where it is desirable to perform a semi-quantitative analysis for any given test sample, the assays and methods described herein may be performed in the absence of test sample to first establish a reference threshold (e.g. in presence of ethanol acting as a negative control). Assay results obtained from a test sample may be then be compared to the reference threshold, to determine the absolute activity attributable to the ligand(s) present in a sample using a simple subtraction methodology.

The present disclosure further contemplates the use of the assays and test kits described herein to determine the potency of a test compound relative to a reference compound. According to the present disclosure, the term 'relative potency' is defined as the multiplier of biological activity of a test compound relative to a reference compound, as determined by normalizing the biological activity of the test compound to the reference compound.

The biological activity of the test and reference compounds may be determined using EC₅₀ or the concentration of compound that gives half the maximal response from a dose response curve for that particular compound. The dose response curve is generated by serially diluting the compound and measuring its steroid hormone receptor binding/activation profile. A plot of the measured activity (e.g. as measured by fluorescence,) vs concentration of the compound (i.e. serial dilution of the compound generates a concentration range that is best presented on a log scale) is then made.

A person skilled in the art will recognize that a measure of relative potency of a test compound is *relative* to the reference compound used. In other words, the relative potency of a test compound is likely to differ depending on the reference compound to which its biological activity is normalized.

Where the relative potency is >1, the test compound invokes a higher measured biological activity in the assays compared to the reference compound. Where the relative potency is <1, the test compound invokes a lower measured biological activity in the assays compared to the reference compound. Where the relative potency =1, the test compound and the reference compound invoke equal biological activity in the assays.

Relative potency can also be used to determine the activation factor of a test compound in question. An activation factor >1 means that the test compound has undergone metabolic conversion to a more physiologically active state in the presence of metabolic machinery in the assay.

Yet another advantage conferred by the test kits and assays according to the present disclosure is the relative ease of performance. In other words, performance of the test kits, assays and assay methods described herein does not require complex cell culture techniques, experienced laboratory technicians or convoluted laboratory testing equipment and analysis. This is particularly advantageous, because the test kits, assays and methods according to the present disclosure may be practiced by untrained personnel in the field following relatively simple testing procedures. Further, performance of the test kits, assays and methods may provide real time information (e.g.) when testing for performance enhancing substances in a sample taken from an athlete immediately prior to, or following, competition.

In other aspects of the present disclosure there is described a test kit for screening a sample for the presence of a ligand, which ligand is capable of forming a complex with a steroid hormone receptor and eliciting a genomic response when in a cell, the test kit comprising:
(i) an androgen receptor that is capable of forming a receptor-ligand complex with a ligand from the test sample; or
(ii) an estrogen receptor that is capable of forming a receptor-ligand complex with a ligand from the test sample, wherein the estrogen receptor is estrogen receptor alpha or estrogen receptor beta; or
(iii) a progesterone receptor that is capable of forming a receptor-ligand complex with a ligand from the test sample, wherein the progesterone receptor is progesterone receptor A or progesterone receptor B; or
(iv) a mineralocorticoid receptor that is capable of forming a receptor-ligand complex with a ligand from the test sample; or
(v) a glucocorticoid receptor that is capable of forming a receptor-ligand complex with a ligand from the test sample; and
(vi) a nucleic acid reporter construct comprising a hormone response element that is capable of being bound by the receptor-ligand complex;
wherein, the presence of a ligand in the sample is detected by measuring a change in a property of the reporter construct caused by binding of the receptor-ligand complex to the hormone response element when the sample is combined with the test kit.

In further aspects of the present disclosure there is described a test kit for screening a sample for the presence of a ligand, which ligand is capable of forming a complex with a steroid hormone receptor and eliciting a genomic response when in a cell, the test kit comprising:
(i) an androgen receptor that is capable of forming a receptor-ligand complex with a ligand from the test sample; or
(ii) an estrogen receptor that is capable of forming a receptor-ligand complex with a ligand from the test sample, wherein the estrogen receptor is estrogen receptor alpha or estrogen receptor beta; or
(iii) a progesterone receptor that is capable of forming a receptor-ligand complex with a ligand from the test sample, wherein the progesterone receptor is progesterone receptor A or progesterone receptor B; or
(iv) a mineralocorticoid receptor that is capable of forming a receptor-ligand complex with a ligand from the test sample; or
(v) a glucocorticoid receptor that is capable of forming a receptor-ligand complex with a ligand from the test sample; and
(vi) a nucleic acid reporter construct comprising a hormone response element that is capable of being bound by the receptor-ligand complex; and
(vii) heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52,
wherein, the presence of a ligand in the sample is detected by measuring a change in a property of the reporter construct caused by binding of the receptor-ligand complex to the hormone response element when the sample is combined with the test kit.

In other aspects of the present disclosure there is described a test kit for screening a sample for the presence of a ligand, which ligand is capable of forming a complex with a steroid hormone receptor and eliciting a genomic response when in a cell, the test kit comprising:
(i) an androgen receptor that is capable of forming a receptor-ligand complex with a ligand from the test sample; or
(ii) an estrogen receptor that is capable of forming a receptor-ligand complex with a ligand from the test sample, wherein the estrogen receptor is estrogen receptor alpha or estrogen receptor beta; or
(iii) a progesterone receptor that is capable of forming a receptor-ligand complex with a ligand from the test sample, wherein the progesterone receptor is progesterone receptor A or progesterone receptor B; or
(iv) a mineralocorticoid receptor that is capable of forming a receptor-ligand complex with a ligand from the test sample; or
(v) a glucocorticoid receptor that is capable of forming a receptor-ligand complex with a ligand from the test sample; and
(vi) a nucleic acid reporter construct comprising:
   (a) a hormone response element that is capable of being bound by the receptor-ligand complex; and
   (b) a fluorescence generating moiety,
wherein, the presence of a ligand in the sample is detected by measuring a change in fluorescence signal of the reporter construct caused by binding of the receptor-ligand complex to the hormone response element when the sample is combined with the test kit.

In further aspects of the present disclosure there is described a test kit for screening a sample for the presence of a ligand, which ligand is capable of forming a complex with a steroid hormone receptor and eliciting a genomic response when in a cell, the test kit comprising:
(i) an androgen receptor that is capable of forming a receptor-ligand complex with a ligand from the test sample; or
(ii) an estrogen receptor that is capable of forming a receptor-ligand complex with a ligand from the test sample, wherein the estrogen receptor is estrogen receptor alpha or estrogen receptor beta; or
(iii) a progesterone receptor that is capable of forming a receptor-ligand complex with a ligand from the test sample, wherein the progesterone receptor is progesterone receptor A or progesterone receptor B; or
(iv) a mineralocorticoid receptor that is capable of forming a receptor-ligand complex with a ligand from the test sample; or
(v) a glucocorticoid receptor that is capable of forming a receptor-ligand complex with a ligand from the test sample; and
(vi) heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52; and
(vii) a nucleic acid reporter construct comprising:
   (a) a hormone response element that is capable of being bound by the receptor-ligand complex; and
   (b) a fluorescence generating moiety,
wherein, the presence of a ligand in the sample is detected by measuring a change in fluorescence signal of the reporter construct caused by binding of the receptor-ligand complex to the hormone response element when the sample is combined with the test kit.

In further aspects of the present disclosure there is described a test kit for screening a sample for the presence of a ligand, which ligand is capable of forming a complex with a steroid hormone receptor and eliciting a genomic response when in a cell, the test kit comprising:
(i) an androgen receptor that is capable of forming a receptor-ligand complex with a ligand from the test sample; or
(ii) an estrogen receptor that is capable of forming a receptor-ligand complex with a ligand from the test sample, wherein the estrogen receptor is estrogen receptor alpha or estrogen receptor beta; or
(iii) a progesterone receptor that is capable of forming a receptor-ligand complex with a ligand from the test sample, wherein the progesterone receptor is progesterone receptor A or progesterone receptor B; or
(iv) a mineralocorticoid receptor that is capable of forming a receptor-ligand complex with a ligand from the test sample; or
(v) a glucocorticoid receptor that is capable of forming a receptor-ligand complex with a ligand from the test sample; and
(vi) a nucleic acid reporter construct comprising:
   (a) a hormone response element that is capable of being bound by the receptor-ligand complex; and
   (b) a fluorescence generating moiety comprising a quantum dot; and
   (c) a fluorescence quenching moiety comprising a gold nanoparticle,
wherein, the presence of a ligand in the sample is detected by measuring a change in fluorescence signal of the reporter construct caused by binding of the receptor-ligand complex to the hormone response element when the sample is combined with the test kit.

In yet further aspects of the present disclosure there is described a test kit for screening a sample for the presence of a ligand, which ligand is capable of forming a complex with a steroid hormone receptor and eliciting a genomic response when in a cell, the test kit comprising:
(i) an androgen receptor that is capable of forming a receptor-ligand complex with a ligand from the test sample; or
(ii) an estrogen receptor that is capable of forming a receptor-ligand complex with a ligand from the test sample, wherein the estrogen receptor is estrogen receptor alpha or estrogen receptor beta; or
(iii) a progesterone receptor that is capable of forming a receptor-ligand complex with a ligand from the test sample, wherein the progesterone receptor is progesterone receptor A or progesterone receptor B; or
(iv) a mineralocorticoid receptor that is capable of forming a receptor-ligand complex with a ligand from the test sample; or
(v) a glucocorticoid receptor that is capable of forming a receptor-ligand complex with a ligand from the test sample; and
(vi) heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52; and
(vii) a nucleic acid reporter construct comprising:
   (a) a hormone response element that is capable of being bound by the receptor-ligand complex; and
   (b) a fluorescence generating moiety comprising a quantum dot; and
   (c) a fluorescence quenching moiety comprising a gold nanoparticle,
wherein, the presence of a ligand in the sample is detected by measuring a change in fluorescence signal of the reporter construct caused by binding of the receptor-ligand complex to the hormone response element when the sample is combined with the test kit.

In certain examples according to the assays, methods and test kits of the present disclosure, the nucleic acid molecules include one or more copies of various components of the nucleic acid, including the response element or reporter construct. For example, the reporter constructs or the nucleic acid molecules may include a single copy or multiple copies of the nucleic acid response elements including, but not limited to, duplicate copies, triplicate copies, quadruple copies etc.

In another example of the present disclosure, the steroid hormone receptor is purified from a cell, or is derived from a cell-based hormone receptor through recombinant cloning, expression and purification. In a further example, the steroid hormone receptor is synthetic, and its sequence modeled on, or evolved from, endogenous steroid hormone receptor sequences known in the art.

A person skilled in the art would also recognize that any steroid hormone receptor may be employed in the test kits, assays and methods of the present disclosure, provided that it retains the ability to bind to, and be activated by, a ligand of interest for detection. This includes, steroid hormone receptors, based on endogenous cellular forms, as well as recombinant or synthetic forms.

As such, the test kits, assays and methods according to the present disclosure may be configured to screen/detect any ligand that elicits a steroid hormone genomic response. However, a person skilled in the art will recognise that, according to the various assays concepts described herein, detection of different hormone classes (i.e. ligands) requires the format of the test kits, assays and methods to be properly configured and optimized. For example, detection of a ligand that binds to and activates an androgen receptor, such as testosterone as well as other testosterone-like hormones, requires test kits/assays comprising androgen receptor together with an androgen response element capable of binding to an activated androgen-receptor complex etc.

Designer steroids and non-steroidal anabolic drugs pose a significant and growing challenge for anti-doping laboratories. First identified in the early 2000s with the detection of tetrahydrogestrinone and madol, the threat posed by designer anabolic drugs has rapidly increased to include numerous potential agents. These synthetically-derived anabolic drugs are designed to evade detection or legal controls with respect to both manufacture and supply, and many are widely available on the internet where they are sold as so-called "supplements".

Mass spectrometry remains the primary technology for the identification of known illicit steroid hormones and non-steroid anabolic drugs in biological samples and/or supplements. Despite its sensitivity and specificity, mass spectrometry remains limited by requiring prior knowledge of the steroid and non-steroid anabolic drug's chemical structures for detection. Moreover, mass spectrometry fails to provide information about the biological activity of the anabolic drugs detected, and is unable to differentiate between bioactive and inactive molecules. This is information that is required for legal prosecution of athletes, coaches, trainers, managers and manufacturers.

In recent years, yeast and mammalian cell-based *in vitro* androgen bioassays have been used to detect the presence of novel synthetic androgens, the androgenic potential of progestins as well as androgens, pro-androgens, designer androgens and designer non-steroid anabolic drugs in supplements. However, these assays suffer limitations associated with molecular complexity, as described elsewhere herein, and require technical skills that are both molecular and cellular in nature, are time consuming, labour intensive and expensive. As such, it is not feasible to consider the assays in their present form for inclusion in routine screening. In other words, yeast and mammalian cell-based assays suffer significant limitations because they are not high throughput or cost effective.

Advantageously, the present disclosure describes activity based test kits, assays and methods that work fundamentally on the principle of steroid hormone receptor activation. By detecting steroid hormone receptor activation by a ligand present within a sample to be tested, the present disclosure provides cell-free test kits, assays and methods that do not rely on structural knowledge of the ligand(s) being interrogated, can readily distinguish between the presence of biologically active and inactive ligands, biologically active agonists or antagonists, and provide cost-effective, reliable and reproducible systems that do not require complex laboratory equipment or particular expertise to perform.

Accordingly, in an example according to the test kits, assays and methods described herein, the ligand is a performance enhancing designer drug and/or steroid.

In another example according to the test kits, assays and methods described herein, the ligand is of an unknown chemical structure.

In a further example according to the test kits, assays and methods described herein, the ligand is of a previously unknown chemical structure.

The present disclosure further contemplates use of the test kits, assays and methods as described herein for detecting antagonists of a target ligand by screening a sample of interest for a compound that will prevent binding of the ligand to its steroid hormone receptor such that it no longer activates the receptor and elicits a genomic response.

This is particularly useful when there is a need to screen for antagonists that block steroid hormone receptor activation (e.g.) as potential therapeutics for the treatment of endocrine and non-endocrine cancers. For example, the activity based test kits, methods and assays comprising one or more estrogen receptors according to the present disclosure can be used to screen compound libraries for the presence of antagonists or to monitor the loss of estrogen receptor activation in breast cancer tissue or blood in patients on cancer therapy.

In yet a further example according to all aspects of the test kits, assays and methods described herein, the biological sample is derived from an animal selected from the group consisting of equine, canine, camelid, bovine, porcine, ovine, caprine, avian, simian, murine, leporine, cervine, piscine, salmonid, primate and human.

In yet a further example according to all aspects of the test kits, assays and methods described herein, the test sample is derived from biological material selected from the group consisting of urine, saliva, stool, hair, tissues including, but not limited to, blood (plasma and serum), muscle, tumors, semen, etc.

In yet a further example according to all aspects of the test kits, assays and methods described herein, the test sample is derived from a food selected from the group consisting of vegetable, meat, beverage including but not limited to sports drink and milk, supplements including, but not limited to, food supplements and sports supplements, nutritional supplements, herbal extracts, etc.

In yet a further example according to all aspects of the test kits, assays and methods described herein, the test sample is derived from a medication selected from the group consisting of drug, tonic, syrup, pill, lozenge, cream, spray and gel.

In yet a further example according to all aspects of the test kits, assays and methods described herein, the sample is derived from the environment selected from the group consisting of liquid, water, soil, textile including, but not limited to, plastics and mineral.

In another example, the sample is a biological sample. In a related example, the biological sample is a body fluid sample, including but not limited to, blood, plasma, serum, saliva, interstitial fluid, semen and urine.

In another example, the sample derived from a plant, including but not limited to, leaf, flower, stem, bark, root, bud, pod, pollen and seed.

In another example, the sample is derived from an animal including, but not limited to, an equine animal, a canine animal, a dromedary animal, a bovine animal, a porcine animal, an ovine animal, a caprine animal, an avian animal, a simian animal, a murine animal, a leporine animal, a cervine animal, a piscine animal, a salmonid animal, a primate animal, and a human animal.

In another example, the test sample is a non-biological sample. In a related example, the non-biological sample includes, but is not limited to, a liquid sample including water, a soil sample, a textile sample including but not limited to plastics, a mineral sample, a food sample and a medication.

Examples of a food sample includes, but is not limited to, vegetables, meats, beverages, supplements and herbal extracts.

Examples of a medication includes, but is not limited to, drugs, tonics, syrups, pills, lozenges, creams, sprays and gels.

The disclosure is further described with reference to the following examples. It will be appreciated that the disclosure is not intended to be limited in any way by these examples.

### EXAMPLES

The information and data which follows demonstrates various prototype assays with respect to the detection of ligands that bind to and activate Androgen Receptor including (e.g.) Testosterone and Dihydrotestosterone, or ligands that bind to and activate Estrogen Receptors including (e.g.) Estradiol. These Examples are used to illustrate the activity assay platform described herein, where the assay concepts and principles exemplified by the detection of ligands that bind to an Androgen Receptor or ligands that bind to Estrogen Receptors (i.e. ER-α and ER-β) would apply equally to the detection of other receptor ligands of interest including, without limitation, ligands that bind to Progesterone Receptor including but not limited to Progesterone, ligands that bind to Mineralocorticoid Receptor including but not limited to Aldosterone, and ligands that bind to the Glucocorticoid Receptor including but not limited to Cortisol.

### EXAMPLE 1

### FRET ASSAY PROTOTYPEs 1 & 2: ASSAY ARCHITECTURE & RESULTS

### 1.1 Assay Architecture: Ligand-SHR/HSP90 & HRE interaction with Fluorescence Resonance Energy Transfer (FRET) Readout

The concept of FRET Assay Prototype 1 and FRET Assay Prototype 2 is that a short double-stranded DNA fragment is labelled at the 5' end with fluorophore Cy3 (or equivalent) and at the 3' end with fluorophore Cy5 (or equivalent). If the double-stranded DNA fragment is short enough to allow Cy3 and Cy5 interaction, Cy3 upon excitation at 540 nm wavelength will pass electrons to Cy5 and Cy5 will then emit energy at 680nm. The assay exploits this chemistry by encoding a hormone response element in the double-stranded DNA such that when the steroid hormone receptor is present in a reaction mix and is activated by its specific class of steroid hormone ligand(s), it will bind to the hormone response element. In this position, the steroid hormone receptor protein will physically block electron transfer from Cy3 to Cy5. This will be measured by a decrease in a 540/680 nm fluorescence readout.

So, in short, two complementary DNA oligonucleotides are labeled with Cy3 and Cy5, respectively.

When the oligonucleotides are not annealed, Cy3 is excited at 540 nm with emission of light at 590nm but not at 680nm. Cy5, on the other hand, does not emit light at 590nm or 680nm, as it is not excited by 540 nm.

When the oligonucleotides are annealed, the close proximity of Cy3 and Cy5 allows fluorescence energy transfer to occur. This results in the emission of light at 680nm, when the annealed probe is excited at 540nm.

The E_{FRET} is calculated from the donor (Cy3) and acceptor (Cy5) channels so 540nm for the donor (excitation) and 680nm (Cy5) for the acceptor (emission).

### 1.2 Length of Oligonucleotides

The hormone response element must be a minimum of 15 bp in length, with a 6 base pair motif followed by 3 base pairs followed by a second 6 base pair motif.

Studies suggest that base pairs either upstream or downstream of these HREs can enhance steroid hormone receptor binding to the response element (e.g. for Estrogen Receptor). Further to this, tandem or multiple hormone response elements have been shown to be additive in the steroid hormone response.

Accordingly, oligonucleotides of 15bp length (minimum), 16bp length (i.e. an additional base pair on 3' end of the oligonucleotide), 17bp length (i.e. an additional base pair at either end of the oligonucleotide) and 21bp length (i.e. towards having a tandem site that would need 30 bp) were tested.

The oligonucleotides encoded for an androgen response element (ARE) for the 15, 16 and 21 bp oligonucleotides, or 17 bp for the estrogen response element.

Both AR and ERa have been used as example SHRs in the following series of experiments.

Figure 1 shows that 15 bp was optimal for generation of FRET, with increasing lengths showing significantly less FRET between Cy3 and Cy5.

### 1.3 DNA concentration of E_{FRET} reaction

The success of FRET Assay Prototypes 1 & 2 depends on the ability to measure decreased E_{FRET} between Cy3 and Cy5. Specifically, FRET Assay Prototype 1 depends on an activated Androgen Receptor binding to an Androgen response element, and physically inhibiting the energy transfer between Cy3 and Cy5, and the subsequent change in fluorescence being detectable using a standard fluorescence reader. First, the concentration range of DNA that led to measurable E_{FRET} was determined and then within this range where change in E_{FRET} (ΔE_{FRET}) could be easily detected.

Figure 2 shows the concentration range of DNA where E_{FRET} output could be detected. Figure 3 then interrogates within this range of DNA, the concentration that allows changes in E_{FRET} to be easily measured. To generate an E_{FRET} reaction whereby there would be a measurable decrease that could be controlled, two reactions were performed. In both reactions, the 15 bp DNA fragment encoded a restriction enzyme site, NspI. The first reaction included buffer and DNA with no NspI enzyme added, whereas the second reaction included NspI enzyme, buffer and DNA. The uncut reaction is used to show full E_{FRET} whereas the second reaction, whereby the DNA fragment is cut in half displacing Cy3 from Cy5, is used to show a decrease in E_{FRET} (ΔE_{FRET}).

Figure 3 shows that for a 15 bp double-stranded DNA fragment, a range of 10ng to 2ng or 1.075pmol to 214 fmol DNA (53.75 - 10.7 nM), representing a copy number of 6.472e¹¹ to 1.294e¹¹ showed the greatest ΔE_{FRET}. These DNA concentrations thus formed the reaction range for FRET Assay Prototype 1.

### 1.4 Working Example FRET Assay Prototype 1: AR + ARE labelled with Cy3 & Cy5

Three reactions were established with 1.075 pmol (53.75 nM) of DNA containing an androgen response element (ARE; 10ng, 6.472e¹¹ molecules), 454.55 fmol (50ng, 2.737e¹¹ molecules, 22.72 nM) of Androgen Receptor (AR), and 1.11 pmol HSP90 (100ng, 6.69e¹¹ molecules, 55.5 nM) in reaction buffer (20mM HEPES pH 7.9, 100mM KCl, 20% glycerol). These reaction concentrations represent a ratio of AR protein:ARE DNA of 0.423: 1 and HSP90 protein:AR protein of 2.44: 1. To Reaction 1, nothing else was added to the reaction mixture. This is total E_{FRET} for the ARE DNA in the presence of non-activated AR bound by HSP90. To Reaction 2, Testosterone (final concentration 250 µM) was added to activate AR to dissociate from HSP90 and bind to ARE DNA. To Reaction 3, ethanol as a vehicle control was added to the reaction mixture to ensure that the diluent did not non-specifically activate AR. Figure 4 shows that a ΔE_{FRET} was only detected in the reaction to which 250 µM testosterone was added showing that for ΔE_{FRET} there needed to be Testosterone-activation of AR. AR alone did not decrease E_{FRET} (Reaction 1) and the diluent, ethanol (Reaction 3), had no effect on E_{FRET}. The % decrease in E_{FRET} induced by Testosterone-activated AR was measured at 16%.

Following this series of reactions, a subsequent investigation using FRET Assay Prototype 1 was performed holding the AR concentration the same as above, but lowering the ARE DNA concentration to the lower end of the titration range measured in Figures 2 and 3. Three reactions were established with 214 fmol ARE DNA (10.7 nM, 2ng, 1.29e¹¹ molecules), 454.55 fmol (22.7 nM, 50ng, 2.737e¹¹ molecules) of AR, and 1.11 pmol HSP90 (55.5 nM, 100ng, 6.69e¹¹ molecules) in reaction buffer (20mM HEPES pH 7.9, 100mM KCl, 20% glycerol). These reaction concentrations represent a ratio of AR protein:ARE DNA of 2.12:1 and HSP90 protein:AR protein of 2.44: 1. To Reaction 1, nothing else was added to the reaction mixture. This is total E_{FRET} for the ARE DNA in the presence of non-activated AR bound by HSP90. To Reaction 2, Testosterone (250 µM) was added to activate AR to dissociate from HSP90 and bind to ARE DNA. To Reaction 3, ethanol as a vehicle control was added to the reaction mixture to ensure that the diluent did not non-specifically activate AR. Figure 5 shows that a ΔE_{FRET} was only detected in the reaction to which 250 µM testosterone was added showing that for ΔE_{FRET} there needed to be testosterone-activation of AR. AR alone did not decrease E_{FRET} (Reaction 1) and the diluent, ethanol (Reaction 3), had no effect on E_{FRET}. The % decrease in E_{FRET} induced by Testosterone-activated AR was measured at 34%.

### 1.5 Working Example FRET Assay Prototype 2: ER + ERE labelled with Cy3 & Cy5

Another test of FRET Assay Prototype was developed using ERa and an ERE DNA fragment (referred to herein as FRET Assay Prototype 2). Three reactions were established with 189.7 fmol ERE DNA (9.48 nM, 2ng, 1.143e¹¹ molecules), 1.51 pmol (75.5 nM, 50ng, 4.548e¹¹ molecules) of ERα, and 1.11 pmol HSP90 (55.5 nM, 100ng, 6.69e¹¹ molecules) in reaction buffer (20mM HEPES pH 7.9, 100mM KCl, 20% glycerol). These reaction concentrations represent a ratio of ERa protein:ERE DNA of 3.99:1 and HSP90 protein:ERa protein of 2.44:1. To Reaction 1, nothing else was added to the reaction mixture. This is total E_{FRET} for the ERE DNA in the presence of non-activated ERa bound by HSP90. To Reaction 2, Estradiol (5 nM) was added to activate ERα to dissociate from HSP90 and bind to ERE DNA. To Reaction 3, ethanol as a vehicle control was added to the reaction mixture to ensure that the diluent did not non-specifically activate ERα. Figure 6 shows that a ΔE_{FRET} was only detected in the reaction to which estradiol was added showing that for ΔE_{FRET} there needed to be estradiol-activation of ERα. ERα alone did not decrease E_{FRET} (Reaction 1) and the diluent, ethanol (Reaction 3), also had no effect on E_{FRET}. The Estradiol-induced % decrease in E_{FRET} was measured at 15%.

Another test of FRET Assay Prototype 1 was performed using AR and ARE DNA and of FRET Assay Prototype 2 using ERa and ERE DNA. Four reactions were established. The first two reactions comprised reaction buffer (20mM HEPES pH 7.9, 100mM KCl, 20% glycerol) with 4.299 pmol ARE DNA (215 nM, 40ng, 2.589e¹² copies), 454.55 fmol (22.7 nM, 50ng, 2.737e¹¹ molecules) of AR and 1.11 pmol HSP90 (55.5 nM, 100ng, 6.69e¹¹ molecules). These reaction concentrations represent a ratio of AR protein:ARE DNA of 0.106:1 and HSP90 protein:AR protein of 2.44:1. Reactions 3 and 4 comprised reaction buffer (20mM HEPES pH 7.9, 100mM KCl, 20% glycerol) with 3.795 pmol ERE DNA (190 nM, 2.285e¹²), 1.51 pmol (75.5 nM, 50ng, 4.548e¹¹ molecules) of ERa and 1.11 pmol HSP90 (22.7 nM, 100ng, 6.69e¹¹ molecules). These reaction concentrations represent a ratio of ERa protein:ERE DNA of 0.199:1 and HSP90 protein:ERα protein of 2.44:1. To Reaction 1 and 3, nothing else was added to the reaction mixture. This is total E_{FRET} for the ARE DNA or ERE DNA in the presence of non-activated AR or ERa bound by HSP90, respectively. To Reactions 2 and 4, Testosterone (250 µM) or Estradiol (5 nM) was added to activate AR or ERa, respectively to dissociate from HSP90 and bind to their respective Hormone Response Elements.

Figure 7 shows that a ΔE_{FRET} was not detected in these reactions where DNA fragment concentration far exceeded SHR concentration.

**Table 8: Stoichiometry of reaction components versus E_{FRET} readout**

| **Ratio of SHR/HRE** | **ΔE_{FRET}** | **Base pair length** |
|---|---|---|
| 2.12:1 | 34% | 15bp |
| 0.423:1 | 16% | 15bp |
| 0.106:1 | 0% | 15bp |
| 3.99:1 | 15% | 17bp |
| 0.199:1 | 0% | 17bp |

The data presented in Table 8 show that the stoichiometry of reaction components influences E_{FRET}. A greater change in E_{FRET} is determined by higher ratio of SHR:HRE. The data also shows the influence of base pair length on outcome. A longer fragment leads to less E_{FRET} (see Figure 1) thereby losing the dynamic range of ΔE_{FRET}. An optimal ratio of SHR/HRE is >0.423, with better ΔE_{FRET} when the ratio >2. This only applies if the base pair length is 15bp. A ratio of >2, with a 17bp length, dramatically decreases ΔE_{FRET}.

### 1.6 DNA sequences tested in FRET Assay Prototype 1 & FRET Assay Prototype 2

**Table 9: DNA oligonucleotides used in Prototype 1**

| **Name** | **Sequence** | **Lenght(bp)** | **HRE type** |
|---|---|---|---|
| SEQ ID NO: 171 | Cy3-5'-AGAACAGCATGTTCT-3' | 15 | Primary ARE |
| SEQ ID NO: 172 | 3'-TCTTGTCGTACAAGA-5'-Cy5 | 15 | Primary ARE |
| SEQ ID NO: 173 | Cy3-5'-CAGGTCAGCATGACCTG-3' | 17 | Primary ERE |
| SEQ ID NO: 174 | 3'-GACCAGTCGTACTGGAC-5'-Cy5 | 17 | Primary ERE |

### 1.7 FRET Assay Prototype 1 & 2 Discussion

In summary, Applicants demonstrate that the binding of ligand to steroid hormone receptors, thereby activating the steroid hormone receptor to bind to its DNA recognition motif (or hormone response element) on a double-stranded DNA fragment can be detected using a disrupted FRET approach.

The reaction requires a tight stoichiometry balance between the number of DNA molecules and number of steroid hormone receptor molecules. Increasing the DNA molecules to a ratio where the number of DNA molecules exceeds the number of steroid hormone receptor molecules is detrimental to ΔE_{FRET}.

The length of DNA fragment is also an important consideration with respect to detection of ΔE_{FRET}.

The reaction is specific to ligand, and is not able to be induced non-specifically by diluent. As previously mentioned this significantly enhances the specificity of the assay.

The reaction is specific to steroid hormone receptor activation, as inactive steroid hormone receptor (bound by HSP90) is not able to disrupt FRET suggesting there is no non-specific binding of steroid hormone receptor to the hormone response element.

### EXAMPLE 2

### OTHER FLUORESCENCE ASSAY PROTOTYPES

### 2.1 Fluorescence Modulation by AR/ARE Platform

The main components of the fluorescence modulation by AR/ARE platform include a DNA construct that encodes an androgen response element (ARE) and incorporates a fluorescence moiety and a quenching moiety, and combined with recombinant androgen receptor (AR), recombinant heat shock protein 90 (HSP90), and a buffer.

The DNA construct will exhibit a high level of fluorescence modulation when ARE is bound by ligand-AR thereby causing a change in the level of fluorescence.
- The DNA constructs comprising
   ∘ Oligonucleotides incorporating either ARE SEQ ID No. 1 and 2
   ∘ Fluorophore dye and a fluorescence quencher matched to the fluorophore dye
- Commercial recombinant AR
- Commercial recombinant HSP90
- Androgenic ligand (e.g. Testosterone)
- Necessary buffers.

### 2.2 Fluorescence Modulation by AR/ARE

Biochemical studies and the crystal structure of the dimerised AR DNA binding domain bound to the double-stranded ARE DNA show a consensus sequence of 15 nucleotides that includes three non-specified nucleotides in the centre of the binding site are not critical for molecular recognition of ARE by AR.

The sense and antisense ARE consensus sequences (SEQ ID No. 6 and SEQ ID No. 7 respectively) can each be synthesized as single-stranded oligonucleotides, which combined and annealed form the double-stranded ARE DNA construct. Additional bases can be added to each end of either, or both, of the ARE strands to add functionality including: replicate the ARE so that multiple copies can be incorporated in a single construct; provide attachment for fluorophore and/or quencher dyes that avoid sterically hindering the binding of AR to ARE; change the annealing parameters of the double-stranded construct; introduce protein recognition or restriction endonuclease sequence elements; introduce sequence elements capable of forming secondary structures;

Fluorescence and quencher dyes can be attached post-synthetically to the 5'- terminus of an oligonucleotide via an amino- or thiol-linker with a 6-Carbon spacer arm.

Post-synthetic labelling of oligonucleotides at internal sites of the sequence is possible by substituting any thymidine with 5-C6-Amino-2'-deoxythymidine. In addition, all other bases can be substituted if required. All dyes available for 5'- labelling can also be attached internally.

The 3'- end of oligonucleotides are labelled post-synthetically via an amino link.

The fluorophore Cy3 and similar cyanine fluorophores (DyLight 547, DyLight 647, Cy5, Alexa Fluor 647) all share the property of excited-state cis/trans isomerization that leads to dependence of their fluorescence on the local environment. Restricting the rotational freedom of the fluorophore proximal to the protein binding site by applying steric hindrance of a binding protein causes an increases fluorescence intensity of a cyanine fluorophore oligonucleotide.

Thus, the brightness of the Cy3 donor fluorophore is increased upon binding of proteins in close proximity meaning that the dye can be used with or without at quencher.

The nucleotides within the ARE most amenable to fluorophore or quencher substitution are those nucleotides not directly involved in the molecular interaction between ARE and AR or nucleotides added to either the 5' or 3' termini of the consensus sequence oligonucleotides. Additionally, the 3' terminus of the sense strand is a dT nucleotide and is amenable to terminus modification.

### 2.3 Fluorescence Modulation by AR/ARE using a DNA construct that forms stable secondary structure labelled with a fluorophore and a quencher

In the assay, Applicants synthesized two oligonucleotides. The first oligonucleotide, comprises the 15-nucleotide sense ARE consensus sequence, which is flanked by two nucleotide sequences that are complementary to each other enabling a stem-loop structure to be formed by intramolecular hybridization. Located at the 5' and 3' termini of this oligonucleotide are a fluorophore and a quencher, which in the stem-loop structure are juxtaposed and sufficiently proximal to reduce the fluorescence of the fluorophore.

The second sequence comprises the 15-nucleotide antisense ARE consensus sequence.

In the first oligonucleotide, the base composition of the complementary flanking sequences are specified so that when mixed with the second oligonucleotide at selected temperatures the intramolecular stem-loop structure denatures and intermolecular hybridization with the second oligonucleotide enables the formation of a region of double-stranded consensus ARE DNA. The kinetic and thermodynamic transition between the intramolecular stem-loop and the intermolecular double-stranded ARE DNA will be governed by temperature and the relative proportions of each and will naturally reach an equilibrium. The measurement of fluorescence will enable distinguishing the relative proportions of the stem-loop form (low fluorescence) and hybridized form (high fluorescence).

The following dT-fluorescein (fluorophore represented by **F**) and dT-dabcyl (quencher represented by **Q**) substituted single stranded oligonucleotide combinations were synthesized:
(Construct 9; SEQ ID NOs: 19 & 20) 
5′-FATTTTATTTAATATAATGGTACAnnnTGTTCTATTATATTAAATAAAATQ-3′
3′-CCATGTnnnACAAGA-5′
(Construct 10; SEQ ID NOs: 21 & 22) 
5′-FTTTATTTAATATAATGGTACAnnnTGTTCTATTATATTAAATAAAQ-3′
3′-CCATGTnnnACAAGA-5′
(Construct 11; SEQ ID NOs: 23 & 24) 
5′-FATTTAATATAATGGTACAnnnTGTTCTATTATATTAAATQ-3′
3′-CCATGTnnnACAAGA-5′
(Construct 12; SEQ ID NOs: 25 & 26) 
5′-FTAATATAATGGTACAnnnTGTTCTATTATATTAQ-3′
3′-CCATGTnnnACAAGA-5′
(Construct 13; SEQ ID NOs: 27 & 28)

5′-**F**TATAATGGTACAnnnTGTTCTATTATA**Q**-3′
3′-CCATGTnnnACAAGA-5′

When the two oligonucleotides are combined with AR, HSP90, an androgenic ligand and buffer, at selected temperatures, the binding of the ligand-activated AR to the double-stranded ARE DNA shifts the equilibrium from the low fluorescent stem-loop structure to a high fluorescence linearised configuration because the AR bound to the ARE stabilises the intermolecular duplex thereby kinetically blocking the transition back to the low fluorescence stem-loop configuration.

In this assay format, the DNA construct assembles during the assay from its component oligonucleotide parts and is kinetically trapped in a highly fluorescent form by the binding of ligand-activated AR. This permits the amount of ligand-activated AR binding to the ARE to be determined.

### 2.4 Fluorescence Modulation by AR/ARE using a DNA construct labelled with a fluorophore and a quencher

In the assay, Applicants labelled two complementary single-stranded DNA strands with either a fluorophore or quencher, which both encode the ARE double-stranded DNA after annealing, wherein the fluorophore and the quencher are sufficiently proximal to reduce the fluorescence of the construct.

The following dT-fluorescein (fluorophore represented by **F**) and dT-dabcyl (quencher represented by **Q**) substituted single stranded oligonucleotide combinations were synthesized:

| | |
|---|---|
| 5'-GGTACA**F**nnnTGTTCT-3' | (SEQ ID NO: 29) |
| 3'-CCATGT**Q**nnnACAAGA-5' | (SEQ ID NO: 30) |
| 5'-GGTACAnnn**F**TGTTCT-3' | (SEQ ID NO: 33) |
| 3'-CCATGT**Q**nnnACAAGA-5' | (SEQ ID NO: 34) |
| 5'-GGTACAnnn**F**TGTTCT-3' | (SEQ ID NO: 31) |
| 3'-CCATGTnnn**Q**ACAAGA-5' | (SEQ ID NO: 32) |

When the two oligonucleotides are combined with AR, HSP90, an androgenic ligand and buffer, at selected temperatures, the binding of the ligand-activated AR to the double-stranded ARE DNA perturbs the quenching and causes the fluorescence to increase. This permits the amount of ligand-activated AR binding to the ARE to be determined.

## Claims

1. A cell-free test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid reporter construct comprising:
(a) a hormone response element that is capable of being bound by the receptor-ligand complex;
(b) a fluorescence generating moiety; and
(c) a fluorescence quenching moiety,
wherein the hormone response element (a) is located between the fluorescence generating moiety (b) and the fluorescence quenching moiety (c).

2. The test kit according to claim 1 wherein the fluorescence generating moiety comprises Cy3.

3. The test kit according to claim 1 or claim 2 wherein the fluorescence quenching moiety comprises Cy5.

4. The test kit according to any one of claims 1 to 3 wherein the hormone response element is selected from any one of SEQ ID Nos: 1 to 10.

5. The test kit according to any one of claims 1 to 4, further comprising a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52.

6. The test kit according to claim 5, wherein the relative amount of HSP90 to steroid hormone receptor is x:1, where x is the amount of HSP90 and is defined as [1.0 ≤ x ≤ 5.0].

7. The test kit according to any one of claims 1 to 6, wherein the relative amount of steroid hormone receptor to nucleic acid reporter construct is y:1, where y is the amount of steroid hormone receptor and is defined as [1.0 ≤ y ≤ 5.0].

8. The test kit according to any one of claims 1 to 7, wherein the steroid hormone receptor is selected from the group consisting of androgen receptor (AR), estrogen receptor alpha (ER-α), estrogen receptor beta (ER-β), progesterone receptor A (PRA), progesterone receptor A (PRB), mineralocorticoid receptor (MR); and glucocorticoid receptor (GR).

9. The test kit according to any one of claims 1 to 8, wherein the steroid hormone receptor is an endogenous steroid hormone receptor purified from a cell, a recombinant steroid hormone receptor or a synthetic steroid hormone receptor.

10. The test kit according to any one of claims 1 to 9, wherein the reporter construct is selected from:
(i) the test kit is configured to detect a ligand that binds to an androgen receptor and the reporter construct is selected from any one of SEQ ID Nos: 1-70;
(ii) the test kit is configured to detect a ligand that binds to an estrogen receptor and the reporter construct is selected from any one of SEQ ID Nos: 71-100;
(iii) the test kit is configured to detect a ligand that binds to progesterone receptor and the reporter construct is selected from any one of SEQ ID Nos: 101-130;
(iv) the test kit is configured to detect a ligand that binds to a mineralocorticoid receptor and the reporter construct is selected from any one of SEQ ID Nos: 131-160; or
(v) the test kit is configured to detect a ligand that binds to a glucocorticoid receptor and the reporter construct is selected from any one of SEQ ID Nos: 131-160.

11. A cell-free assay method for detecting a ligand in a sample, which ligand is capable of eliciting a steroid hormone genomic response, the method comprising the steps of:
(i) contacting a sample with:
(a) a steroid hormone receptor that forms a receptor-ligand complex with a ligand from the test sample;
(b) optionally, heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52;
(c) a nucleic acid reporter construct comprising:
1. a hormone response element that is bound by the receptor-ligand complex;
2. a fluorescence generating moiety; and
3. a fluorescence quenching moiety,
wherein the hormone response element (1) is located between the fluorescence generating moiety (2) and the fluorescence quenching moiety (3);
(ii) measuring a change in fluorescence of the reporter construct,
wherein, a measured change in the fluorescence of the reporter construct reflects that a ligand has been detected in the sample.

12. The assay method according to claim 11, wherein the change in fluorescence of the reporter construct is measured using static quenching or dynamic quenching.

13. The assay method according to claim 11, wherein the change in fluorescence of the reporter construct is measured using Forster Resonance Energy Transfer.

14. The assay method according to any one of claims 11 to 13 for determining the doping status of an athlete, comprising performing the assay method on a sample obtained from the athlete to ascertain if the sample comprises a ligand sufficient to activate a steroid hormone receptor and cause a change in fluorescence of the reporter construct, wherein a change in fluorescence of the reporter construct provides information about the doping status of the athlete.

15. A method according to claim 14, wherein the athlete is selected from a human athlete, an equine athlete, a canine athlete and a camelid athlete.

## Patentansprüche

1. Zellfreier Testkit zum Screening einer Probe auf das Vorhandensein eines Liganden, der in der Lage ist, eine genomische Steroidhormonreaktion auszulösen, wobei der Testkit umfasst:
(i) einen Steroidhormonrezeptor, der in der Lage ist, einen Rezeptor-Liganden-Komplex mit einem Liganden aus der Probe auszubilden; und
(ii) ein Nukleinsäure-Reporterkonstrukt, umfassend:
(a) ein Hormonreaktionselement, das in der Lage ist, von dem Rezeptor-Liganden-Komplex gebunden zu werden;
(b) einen fluoreszenzerzeugenden Teil; und
(c) einen fluoreszenzlöschenden Teil,
wobei sich das Hormonreaktionselement (a) zwischen dem fluoreszenzerzeugenden Teil (b) und dem fluoreszenzlöschenden Teil (c) befindet.

2. Testkit nach Anspruch 1, wobei der fluoreszenzerzeugende Teil Cy3 umfasst.

3. Testkit nach Anspruch 1 oder 2, wobei der fluoreszenzlöschende Teil Cy5 umfasst.

4. Testkit nach einem der Ansprüche 1 bis 3, wobei das Hormonreaktionselement aus einer der SEQ ID NO: 1 bis 10 ausgewählt ist.

5. Testkit nach einem der Ansprüche 1 bis 4, der ferner einen Steroidhormonrezeptor-CoFaktor umfasst, ausgewählt aus Hitzeschockprotein 90 (HSP90), einem Komplex aus HSP90 und Hitzeschockprotein 70 (HSP70), einem Komplex aus HSP90, HSP70 und Hitzeschockprotein 40 (HSP40), einem Komplex aus HSP90, HSP70, HSP40 und p23, einem Komplex aus HSP90, HSP70, HSP40, p23 und hitzeschockproteinorganisierendem Protein (Hop), einem Komplex aus HSP90, HSP70, HSP40, p23, Hop und 48kD Hip-Protein (Hip), einem Komplex aus HSP90, HSP70, HSP40, p23, Hop, Hip und p60, und einem Komplex aus HSP90, HSP70, HSP40, p23, Hop, Hip, p60 und FKBP52.

6. Testkit nach Anspruch 5, wobei die relative Menge von HSP90 zu Steroidhormonrezeptor x : 1 beträgt, wobei x die Menge von HSP90 ist und als [1,0 ≤ x ≤ 5,0] definiert ist.

7. Testkit nach einem der Ansprüche 1 bis 6, wobei die relative Menge von Steroidhormonrezeptor zu dem Nukleinsäure-Reporterkonstrukt y : 1 ist, wobei y die Menge des Steroidhormonrezeptor ist und als [1,0 ≤ y ≤ 5,0] definiert ist.

8. Testkit nach einem der Ansprüche 1 bis 7, wobei der Steroidhormonrezeptor aus der Gruppe ausgewählt ist, bestehend aus Androgenrezeptor (AR), Östrogenrezeptor alpha (ER-α), Östrogenrezeptor beta (ER-β), Progesteronrezeptor A (PRA), Progesteronrezeptor A (PRB), Mineralokortikoidrezeptor (MR); und Glukokortikoidrezeptor (GR).

9. Testkit nach einem der Ansprüche 1 bis 8, wobei der Steroidhormonrezeptor ein endogener, aus einer Zelle gereinigter Steroidhormonrezeptor, ein rekombinanter Steroidhormonrezeptor oder ein synthetischer Steroidhormonrezeptor ist.

10. Testkit nach einem der Ansprüche 1 bis 9, wobei das Reporterkonstrukt ausgewählt ist aus:
(i) der Testkit ist dazu konfiguriert, einen Liganden zu erkennen, der an einen Androgenrezeptor bindet, und das Reporterkonstrukt ist aus einer der SEQ ID NO: 1-70 ausgewählt;
(ii) der Testkit ist dazu konfiguriert, einen Liganden zu erkennen, der an einen Östrogenrezeptor bindet, und das Reporterkonstrukt ist aus einer der SEQ ID NO: 71-100 ausgewählt;
(iii) der Testkit ist dazu konfiguriert, einen Liganden zu erkennen, der an den Progesteronrezeptor bindet, und das Reporterkonstrukt ist aus einer der SEQ ID NO: 101-130 ausgewählt;
(iv) der Testkit ist dazu konfiguriert, einen Liganden zu erkennen, der an einen Mineralokortikoidrezeptor bindet, und das Reporterkonstrukt ist aus einer der SEQ ID NO: 131-160 ausgewählt; oder
(v) der Testkit ist dazu konfiguriert, einen Liganden zu erkennen, der an einen Glukokortikoidrezeptor bindet, und das Reporterkonstrukt ist aus einer der SEQ ID NO: 131-160 ausgewählt.

11. Zellfreies Assayverfahren zum Erkennen eines Liganden in einer Probe, wobei der Ligand in der Lage ist, eine genomische Steroidhormonreaktion auszulösen, wobei das Verfahren die Schritte umfasst:
(i) Inberührungbringen einer Probe mit:
(a) einem Steroidhormonrezeptor, der einen Rezeptor-Liganden-Komplex mit einem Liganden aus der Testprobe ausbildet;
(b) optional Hitzeschockprotein 90 (HSP90), einem Komplex aus HSP90 und Hitzeschockprotein 70 (HSP70), einem Komplex aus HSP90, HSP70 und Hitzeschockprotein 40 (HSP40), einem Komplex aus HSP90, HSP70, HSP40 und p23, einem Komplex aus HSP90, HSP70, HSP40, p23 und hitzeschockproteinorganisierendem Protein (Hop), einem Komplex aus HSP90, HSP70, HSP40, p23, Hop und 48kD Hip-Protein (Hip), einem Komplex aus HSP90, HSP70, HSP40, p23, Hop, Hip und p60, und einem Komplex aus HSP90, HSP70, HSP40, p23, Hop, Hip, p60 und FKBP52;
(c) einem Nukleinsäure-Reporter-Konstrukt, umfassend
1. ein Hormonreaktionselement, das durch den Rezeptor-Liganden-Komplex gebunden ist;
2. einem fluoreszenzerzeugenden Teil; und
3. einem fluoreszenzlöschenden Teil,
wobei sich das Hormonreaktionselement (1) zwischen dem fluoreszenzerzeugenden Teil (2) und dem fluoreszenzlöschenden Teil (3) befindet;
(ii) Messen einer Änderung der Fluoreszenz des Reporterkonstrukts,
wobei eine gemessene Änderung der Fluoreszenz des Reporterkonstrukts widerspiegelt, das ein Ligand in der Probe erkannt worden ist.

12. Assayverfahren nach Anspruch 11, wobei die Änderung der Fluoreszenz des Reporterkonstrukts unter Verwendung von statischem Löschen oder dynamischem Löschen gemessen wird.

13. Assayverfahren nach Anspruch 11, wobei die Änderung der Fluoreszenz des Reporterkonstrukts unter Verwendung von Forster-Resonanz-Energie-Transfer gemessen wird.

14. Assayverfahren nach einem der Ansprüche 11 bis 13 zum Bestimmen des Dopingstatus eines Athleten, umfassend das Durchführen des Assayverfahrens an einer von dem Athleten erhaltenen Probe, um festzustellen, ob die Probe einen Liganden umfasst, der ausreicht, um einen Steroidhormonrezeptor zu aktivieren und eine Änderung der Fluoreszenz des Reporterkonstrukts zu bewirken, wobei eine Änderung der Fluoreszenz des Reporterkonstrukts Informationen über den Dopingstatus des Athleten bereitstellt.

15. Assayverfahren nach Anspruch 14, wobei der Athlet aus einem menschlichen Athleten, einem Pferdeathleten, einem Hundeathleten und einem Kamelidenathleten ausgewählt ist.

## Revendications

1. Kit de test acellulaire pour le dépistage, dans un échantillon, de la présence d'un ligand capable de déclencher une réponse génomique d'hormone stéroïdienne, le kit de test comprenant :
(i) un récepteur d'hormone stéroïdienne capable de former un complexe récepteur-ligand avec un ligand de l'échantillon, et
(ii) une construction d'acide nucléique rapporteur comprenant :
(a) un élément de réponse hormonale susceptible de subir une liaison au complexe récepteur-ligand,
(b) une fraction génératrice de fluorescence, et
(c) une fraction d'atténuation de fluorescence ;
ledit élément de réponse hormonale (a) étant situé entre la fraction génératrice de fluorescence (b) et la fraction d'atténuation de fluorescence (c).

2. Kit de test selon la revendication 1, dans lequel la fraction génératrice de fluorescence comprend Cy3.

3. Kit de test selon la revendication 1 ou la revendication 2, dans lequel la fraction d'atténuation de fluorescence comprend Cy5.

4. Kit de test selon l'une quelconque des revendications 1 à 3, dans lequel l'élément de réponse hormonale est sélectionné parmi l'une quelconque des séquences de SEQ ID N° : 1 à 10.

5. Kit de test selon l'une quelconque des revendications 1 à 4, comprenant en outre un cofacteur de récepteur d'hormone stéroïdienne sélectionné parmi une protéine de choc thermique 90 (HSP90), un complexe de HSP90 et de protéine de choc thermique 70 (HSP70), un complexe de HSP90, HSP70 et de protéine de choc thermique 40 (HSP40), un complexe de HSP90, HSP70, HSP40 et p23, un complexe de HSP90, HSP70, HSP40, p23 et de protéine organisatrice de protéines de choc thermique (Hop), un complexe de HSP90, HSP70, HSP40, p23, Hop et de protéine Hip de 48 kD (Hip), un complexe de HSP90, HSP70, HSP40, p23, Hop, Hip et p60, et un complexe de HSP90, HSP70, HSP40, p23, Hop, Hip, p60 et FKBP52.

6. Kit de test selon la revendication 5, dans lequel la quantité relative de HSP90 par rapport au récepteur d'hormone stéroïdienne est de x : 1, où x est la quantité de HSP90 définie sous la forme [1,0 ≤ x ≤ 5,0].

7. Kit de test selon l'une quelconque des revendications 1 à 6, dans lequel la quantité relative de récepteur d'hormone stéroïdienne par rapport à la construction d'acide nucléique rapporteur est de y : 1, où y est la quantité de récepteur d'hormone stéroïdienne définie sous la forme [1,0 ≤ y ≤ 5,0].

8. Kit de test selon l'une quelconque des revendications 1 à 7, dans lequel le récepteur d'hormone stéroïdienne est sélectionné dans le groupe constitué de récepteur des androgènes (AR), récepteur d'oestrogène alpha (ER-α), récepteur d'oestrogène bêta (ER-β), récepteur de progestérone A (PRA), récepteur de progestérone A (PRB), récepteur des minéralocorticoïdes (MR) ; et récepteur des glucocorticoïdes (GR).

9. Kit de test selon l'une quelconque des revendications 1 à 8, dans lequel le récepteur d'hormone stéroïdienne est un récepteur d'hormone stéroïdienne endogène purifié à partir d'une cellule, un récepteur d'hormone stéroïdienne recombinant ou un récepteur d'hormone stéroïdienne synthétique.

10. Kit de test selon l'une quelconque des revendications 1 à 9, dans lequel la construction de rapporteur est sélectionnée parmi les suivantes :
(i) le kit de test est conçu pour détecter un ligand qui se lie à un récepteur des androgènes et la construction de rapporteur est sélectionnée parmi l'une quelconque des séquences de SEQ ID N° : 1 à 70 ;
(ii) le kit de test est conçu pour détecter un ligand qui se lie à un récepteur d'oestrogène et la construction de rapporteur est sélectionnée parmi l'une quelconque des séquences de SEQ ID N° : 71 à 100 ;
(iii) le kit de test est conçu pour détecter un ligand qui se lie à un récepteur de progestérone et la construction de rapporteur est sélectionnée parmi l'une quelconque des séquences de SEQ ID N° : 101 à 130 ;
(iv) le kit de test est conçu pour détecter un ligand qui se lie à un récepteur des minéralocorticoïdes et la construction de rapporteur est sélectionnée parmi l'une quelconque des séquences de SEQ ID N° : 131 à 160 ; ou
(v) le kit de test est conçu pour détecter un ligand qui se lie à un récepteur des glucocorticoïdes et la construction de rapporteur est sélectionnée parmi l'une quelconque des séquences de SEQ ID N° : 131 à 160.

11. Procédé de dosage acellulaire pour détecter un ligand dans un échantillon, ledit ligand étant capable de déclencher une réponse génomique d'hormone stéroïdienne, le procédé comprenant les étapes suivantes :
(i) mise en contact d'un échantillon avec :
(a) un récepteur d'hormone stéroïdienne qui forme un complexe récepteur-ligand avec un ligand de l'échantillon testé,
(b) éventuellement, une protéine de choc thermique 90 (HSP90), un complexe de HSP90 et de protéine de choc thermique 70 (HSP70), un complexe de HSP90, HSP70 et de protéine de choc thermique 40 (HSP40), un complexe de HSP90, HSP70, HSP40 et p23, un complexe de HSP90, HSP70, HSP40, p23 et de protéine organisatrice de protéines de choc thermique (Hop), un complexe de HSP90, HSP70, HSP40, p23, Hop et de protéine Hip de 48 kD (Hip), un complexe de HSP90, HSP70, HSP40, p23, Hop, Hip et p60, et un complexe de HSP90, HSP70, HSP40, p23, Hop, Hip, p60 et FKBP52,
(c) une construction d'acide nucléique rapporteur comprenant :
1. un élément de réponse hormonale qui subit une liaison au complexe récepteur-ligand,
2. une fraction génératrice de fluorescence, et
3. une fraction d'atténuation de fluorescence,
ledit élément de réponse hormonale (1) étant situé entre la fraction génératrice de fluorescence (2) et la fraction d'atténuation de fluorescence (3) ;
(ii) mesure d'un changement de fluorescence de la construction de rapporteur,
la mesure d'un changement de fluorescence de la construction de rapporteur indiquant qu'un ligand a été détecté dans l'échantillon.

12. Procédé de dosage selon la revendication 11, dans lequel le changement de fluorescence de la construction de rapporteur est mesuré à l'aide d'une atténuation statique ou d'une atténuation dynamique.

13. Procédé de dosage selon la revendication 11, dans lequel le changement de fluorescence de la construction de rapporteur est mesuré à l'aide d'un transfert d'énergie par résonance de Forster.

14. Procédé de dosage selon l'une quelconque des revendications 11 à 13 pour déterminer l'état de dopage d'un athlète, comprenant la réalisation du procédé de dosage sur un échantillon obtenu auprès de l'athlète pour vérifier si l'échantillon comprend un ligand suffisant pour activer un récepteur d'hormone stéroïdienne et entraîner un changement de fluorescence de la construction de rapporteur, étant entendu qu'un changement de fluorescence de la construction de rapporteur fournit des informations sur l'état de dopage de l'athlète.

15. Procédé selon la revendication 14, dans lequel l'athlète est sélectionné parmi un athlète humain, un athlète équin, un athlète canin et un athlète camélidé.
